# EUROPEAN PATENT APPLICATION

(11) **EP 4 534 019 A1**
(43) Date of publication of application: **09.04.2025**
(21) Application number: 24188088.9
(22) Date of filing: 11.07.2024
(51) Int. Cl.: A61B 8/08, A61B 8/00

(54) **ULTRASOUND IMAGING DEVICE AND CONTROL METHOD THEREOF**

(30) Priority: 06.10.2023 KR 20230133631; 28.02.2024 KR 20240029220
(71) Applicant: Samsung Medison Co., Ltd., Hongcheon-gun, Gangwon-do 25108 (KR); Yonsei University, University-Industry Foundation(UIF)., Seoul 03722 (KR)
(72) Inventor: JO, Hyeoncheol, 05340 Seoul (KR); KIM, Hanjun, 05340 Seoul (KR); JANG, Taejun, 05340 Seoul (KR); KWON, Ja-young, 03722 Seoul (KR); KWON, Hayan, 03722 Seoul (KR); JUNG, Yunji, 03722 Seoul (KR); HUR, Hyewon, 03722 Seoul (KR)
(74) Representative: Frey, Sven Holger

(57) **Abstract**

A method of controlling an ultrasound imaging device is disclosed. The method may include obtaining volume data of an object, obtaining a reference cross-sectional image crossing a first axis from the volume data, obtaining, from the volume data, a candidate standard cross-sectional image crossing a second axis that is different from the first axis, displaying the reference cross-sectional image and the candidate standard cross-sectional image on a first graphic user interface (GUI) view, rotating the reference cross-sectional image on the first GUI view, updating the candidate standard cross-sectional image on the first GUI view by adjusting a slicing plane of the candidate standard cross-sectional image based on the rotation of the reference cross-sectional image, and displaying, according to completion of the rotation of the reference cross-sectional image, the updated candidate standard cross-sectional image as a standard cross-sectional image on the first GUI view.

## Description

### BACKGROUND

### 1. Field

The disclosure relates to an ultrasound imaging device and a control method thereof. More specifically, the disclosure relates to an ultrasound imaging device for updating a standard cross-sectional image and a control method thereof.

### 2. Description of the Related Art

Recently, in the medical field, various medical imaging devices have been widely used to image and obtain information about biological tissues of the human body for the purpose of early diagnosis of various diseases or surgery. Representative examples of the medical imaging devices include ultrasound imaging devices, computed tomography (CT) devices, and magnetic resonance imaging (MRI) devices.

The ultrasound imaging device is an apparatus that irradiates an ultrasound signal generated from a transducer of a probe to an object and receives information of the signal reflected from the object to non-invasively obtain at least one image about an internal area (for example, soft tissue or blood flow) of the object. The ultrasound imaging device can be used for medical purposes, such as observing the interior of an object, detecting foreign materials, and measuring injuries. Advantages of the ultrasound imaging devices include having higher stability than imaging devices using X rays, displaying images in real time, and being safe as there is no radiation exposure. Therefore, the ultrasound imaging devices are widely used together with other imaging devices.

### SUMMARY

Additional aspects will be set forth in part in the description which follows and, in part, will be apparent from the description, or may be learned by practice of the presented embodiments of the disclosure.

A method of controlling an ultrasound imaging device, according to an embodiment of the disclosure, may include obtaining a reference cross-sectional image crossing a first axis from volume data. In an embodiment, the method of controlling the ultrasound imaging device may include obtaining, from the volume data, a candidate standard cross-sectional image crossing a second axis that is different from the first axis. In an embodiment, the method of controlling the ultrasound imaging device may include displaying the reference cross-sectional image and the candidate standard cross-sectional image on a first Graphic User Interface (GUI) view. In an embodiment, the method of controlling the ultrasound imaging device may include rotating the reference cross-sectional image on the first GUI view. In an embodiment, the method of controlling the ultrasound imaging device may include updating the candidate standard cross-sectional image on the first GUI view by adjusting a slicing plane of the candidate standard cross-sectional image based on the rotation of the reference cross-sectional image. In an embodiment, the method of controlling the ultrasound imaging device may include displaying, according to completion of the rotation of the reference cross-sectional image, the updated candidate standard cross-sectional image as a standard cross-sectional image on the first GUI view. In an embodiment, the standard cross-sectional image may include at least one anatomical landmark.

An ultrasound imaging device according to an embodiment of the disclosure may include a display, a memory storing one or more instructions, and at least one processor configured to execute the one or more instructions stored in the memory. In an embodiment, the at least one processor may be configured to execute the one or more instructions to obtain a reference cross-sectional image crossing a first axis from volume data. In an embodiment, the at least one processor may be configured to execute the one or more instructions to obtain, from the volume data, a candidate standard cross-sectional image crossing a second axis that is different from the first axis. In an embodiment, the at least one processor may be configured to execute the one or more instructions to display the reference cross-sectional image and the candidate standard cross-sectional image on a first Graphic User Interface (GUI) view. In an embodiment, the at least one processor may be configured to execute the one or more instructions to rotate the reference cross-sectional image on the first GUI view. updating the candidate standard cross-sectional image on the first GUI view by adjusting a slicing plane of the candidate standard cross-sectional image based on the rotation of the reference cross-sectional image. In an embodiment, the at least one processor may be configured to execute the one or more instructions to display, according to completion of the rotation of the reference cross-sectional image, the updated candidate standard cross-sectional image as a standard cross-sectional image on the first GUI view. In an embodiment, the standard cross-sectional image may include at least one anatomical landmark.

According to an embodiment of the disclosure, a computer-readable recording medium storing a program for performing at least one of the method of controlling the ultrasound imaging device on a computer may be provided.

### BRIEF DESCRIPTION OF THE DRAWINGS

The above and other aspects, features, and advantages of certain embodiments of the disclosure will be more apparent from the following description taken in conjunction with the accompanying drawings.

The disclosure may be easily understood by combining the following detailed description with the accompanying drawings, and reference numerals represent structural elements.
FIGS. 1A and 1B are block diagrams showing configurations of an ultrasound imaging system according to an embodiment of the disclosure;
FIGS. 2A, 2B, 2C, and 2D show an ultrasound imaging system according to an embodiment of the disclosure;
FIGS. 3A and 3B are block diagrams showing configurations of an ultrasound imaging device according to an embodiment of the disclosure;
FIG. 4 is a flowchart for describing a method of controlling an ultrasound imaging device, according to an embodiment of the disclosure;
FIG. 5A is a view for describing a reference cross-sectional image and a standard cross-sectional image according to an embodiment of the disclosure;
FIG. 5B is a view for describing a candidate standard cross-sectional image and a standard cross-sectional image according to an embodiment of the disclosure;
FIG. 6 is a view for describing a first graphic user interface (GUI) according to an embodiment of the disclosure;
FIGS. 7A and 7B are views for describing an update of a candidate standard cross-sectional image according to an embodiment of the disclosure;
FIGS. 8A and 8B are views for describing displaying of at least one anatomical landmark with a preset color by an ultrasound imaging device according to an embodiment of the disclosure; and
FIG. 9 is a view for describing displaying of a three-dimensional ultrasound image by an ultrasound imaging device according to an embodiment of the disclosure.

### DETAILED DESCRIPTION

The present specification will describe the principles of embodiments of the disclosure and disclose the embodiments to clarify the scope of rights of the claims of the disclosure and for those skilled in the technical art to which the embodiments of the disclosure pertain to embody the embodiments of the disclosure. The embodiments of the disclosure may be implemented in various forms.

Throughout this specification, like reference numerals will refer to like components. The present specification does not describe all elements of the embodiments, and descriptions about content being general in the technical art to which the disclosure pertains or overlapping content between the embodiments will be omitted. As used herein, the terms "module" or "unit" may be implemented as one or a combination of two or more of software, hardware, or firmware, and according to some embodiments, a plurality of "modules" or "units" may be implemented as a single element, or a single "module" or "unit" may include a plurality of elements.

The singular form of a noun corresponding to an item may include one or a plurality of the items unless clearly indicated otherwise in a related context.

In the disclosure, phrases, such as "A or B", "at least one of A and B", "at least one of A or B," "A, B or C," "at least one of A, B and C," and "at least one of A, B, or C", may include any one or all possible combinations of items listed together in the corresponding phrase among the phrases.

The term "and/or" includes any or all combinations of a plurality of associated listed components.

Terms such as "first", "second", or "1st" or "2nd" may be used simply to distinguish a component from other components, without limiting the component in other aspects (e.g., importance or order).

Also, the terms 'front surface', 'rear surface', 'upper surface', 'lower surface', 'side surface', 'left side', 'right side', 'upper portion', 'lower portion', etc., as used in the disclosure, are defined based on the drawings, and the shapes and positions of the components are not limited by the terms.

It will be understood that when the terms "includes," "comprises," "including," and/or "comprising," when used in the disclosure, specify the presence of stated features, figures, steps, operations, components, members, or combinations thereof, but do not preclude the presence or addition of one or more other features, figures, steps, operations, components, members, or combinations thereof.

It will be understood that when a certain component is referred to as being "connected to", "coupled to", "supported by" or "in contact with" another component, it can be directly or indirectly connected to, coupled to, supported by, or in contact with the other component. When a component is indirectly connected to, coupled to, supported by, or in contact with another component, it may be connected to, coupled to, supported by, or in contact with the other component through a third component.

It will also be understood that when a certain component is referred to as being "on" or "over" another component, it can be directly on the other component or intervening components may also be present.

Hereinafter, an ultrasound imaging device according to various embodiments will be described in detail with reference to the accompanying drawings. In the following description with reference to the accompanying drawings, the same or corresponding components are assigned similar reference numerals, and overlapping descriptions thereof may be omitted.

In the disclosure, an image may include a medical image obtained by a medical imaging apparatus, such as a magnetic resonance imaging (MRI) apparatus, a computed tomography (CT) apparatus, an ultrasound imaging apparatus, an X-ray imaging apparatus, and the like.

In the disclosure, 'object' may be a target to be photographed, and include a human, an animal, or a part thereof. For example, an object may include a part (intestines, organs, etc.) of a body, a phantom, etc.

In the disclosure, an "ultrasound image" means an image about an object generated or processed based on an ultrasound signal transmitted to an object and then reflected from the object.

Hereinafter, embodiments of the disclosure will be described in detail with reference to the accompanying drawings.

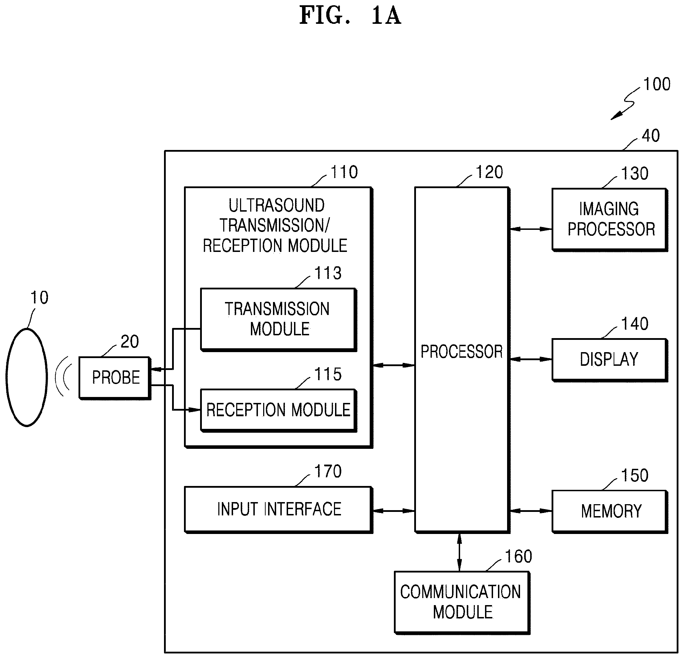

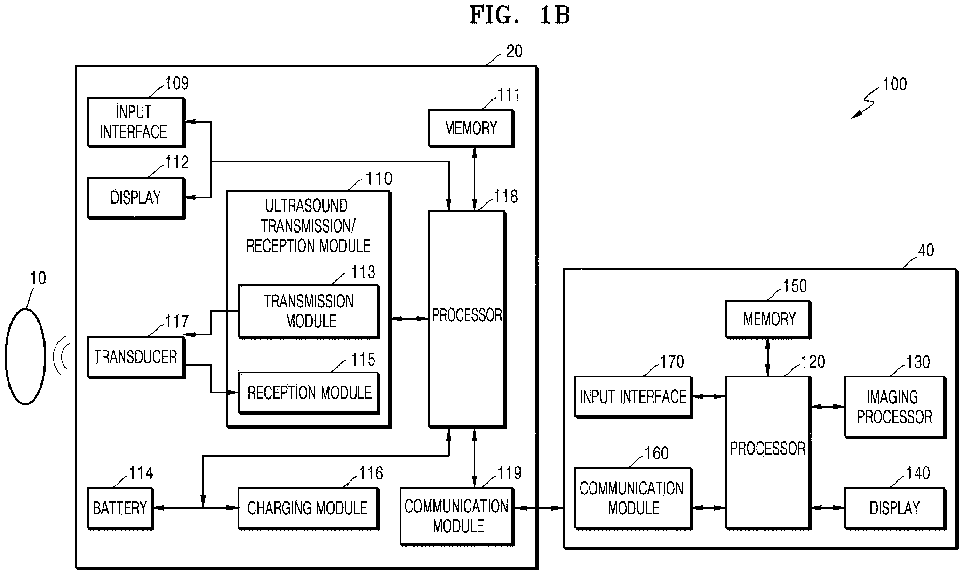

FIGS. 1A and 1B are block diagrams showing configurations of an ultrasound imaging system according to an embodiment of the disclosure.

Referring to FIGS. 1A and 1B, an ultrasound imaging system 100 may include a probe 20 and an ultrasound imaging device 40.

The ultrasound imaging device 40 may be implemented as a cart type or a portable type. Examples of portable ultrasound imaging devices may include a smart phone, a laptop computer, a personal digital assistant (PDA), a tablet PC, etc., each of which includes a probe and an application, although not limited thereto. The ultrasound imaging device 40 may be implemented as a probe-integrated type.

The probe 20 may include a wired probe connected to the ultrasound imaging device 40 by wire to communicate with the ultrasound imaging device 40 by wire, a wireless probe wirelessly connected to the ultrasound imaging device 40 to wirelessly communicate with the ultrasound imaging device 40, and/or a hybrid probe connected to the ultrasound imaging device 40 by wire or wirelessly to communicate with the ultrasound imaging device 40 by wire or wirelessly.

According to various embodiments of the disclosure, as shown in FIG. 1A, the ultrasound imaging device 40 may include an ultrasound transmission/reception module 110, or the probe 20 may include the ultrasound transmission/reception module 110 as shown in FIG. 1B. According to various embodiments of the disclosure, both the ultrasound imaging device 40 and the probe 20 may include the ultrasound transmission/reception module 110.

According to various embodiments of the disclosure, the probe 20 may include at least one of an imaging processor 130, a display 140, or an input interface 170, or a combination thereof. In the disclosure, descriptions about the ultrasound transmission/reception module 110, the imaging processor 130, the display 140, or the input interface 170, included in the ultrasound imaging device 40 may be applied to the ultrasound transmission/reception module 110, the imaging processor 130, the display 140, or the input interface 170, included in the probe 20.

FIG. 1A is a block diagram showing a configuration of the ultrasound imaging system 100 with the probe 20 which is a wired probe or a hybrid probe.

The probe 20 may include a plurality of transducers. The plurality of transducers may be arranged in a preset arrangement and implemented as a transducer array. The transducer array may correspond to a one-dimensional (1D) array or a two-dimensional (2D) array. The plurality of transducers may transmit an ultrasound signal to an object 10 according to a transmission signal applied from a transmission module 113. The plurality of transducers may form a reception signal by receiving an ultrasound signal (an echo signal) reflected from the object 10. Also, the probe 20 may be implemented as an integrated type integrated into the ultrasound imaging device 40, or a separated type connected to the ultrasound imaging device 40 by wire. Also, the ultrasound imaging device 40 may be connected to a single or plurality of probes 20 according to an implementation type.

In the case in which the probe 20 is a wired probe or a hybrid probe, the probe 20 may include a cable and a connector that are connectable to a connector of the ultrasound imaging device 40.

The probe 20 according to an embodiment of the disclosure may be implemented as a two-dimensional probe. In the case in which the probe 20 is a two-dimensional probe, the plurality of transducers included in the probe 20 may form a two-dimensional transducer array by being arranged two-dimensionally.

For example, the two-dimensional transducer array may be configured by arranging a plurality of sub arrays each including a plurality of transducers arranged in a first direction in a second direction that is different from the first direction.

Also, in the case in which the probe 20 according to an embodiment of the disclosure is a two-dimensional probe, the ultrasound transmission/reception module 110 may include at least one of an analog beamformer or a digital beamformer. Also, according to an embodiment of the disclosure, the two-dimensional probe may include at least one of an analog beamformer or a digital beamformer, or a combination thereof according to an implementation type.

The processor 120 may control the transmission module 113 to form a transmission signal that is to be applied to each of a plurality of transducers 117 included in the probe 20 in consideration of positions and focus points of the transducers 117.

The processor 120 may control the reception module 115 to perform analog-to-digital conversion on reception signals received from the probe 20, sum the digital-converted reception signals in consideration of the positions and focus points of the plurality of transducers 117, and thereby generate ultrasound data.

In the case in which the probe 20 is implemented as a two-dimensional probe, the processor 120 may calculate a time delay value for digital beamforming for each of a plurality of sub arrays included in a two-dimensional transducer array. Also, the processor 120 may calculate a time delay value for analog beamforming for each of transducers included in any sub array among the plurality of sub arrays. The processor 120 may control the analog beamformer and the digital beamformer to form a transmission signal that is to be applied to each of the plurality of transducers according to time delay values for analog beamforming and time delay values for digital beamforming. Also, the processor 120 may control the analog beamformer to sum signals received from the plurality of transducers for each sub array according to the time delay values for analog beamforming. Also, the processor 120 may control the ultrasound transmission/reception module 110 to perform analog-to-digital conversion on signals summed for the respective sub arrays. Also, the processor 120 may control the digital beamformer to sum the digital-converted signals according to the time delay values for digital beamforming and generate ultrasound data.

The imaging processor 130 may generate or process an ultrasound image by using the generated ultrasound data.

The display 140 may display the generated ultrasound image and various information processed in the ultrasound imaging device 40 or the probe 20. The probe 20 or the ultrasound imaging device 40 may include a single or plurality of displays 140 according to an implementation type. Also, the display 140 may include a touch panel or a touch screen. Also, the display 140 may include a flexible display.

The processor 120 may control overall operations of the ultrasound imaging device 40, and control operations of components of the ultrasound imaging device 40. The processor 120 may execute a program or instructions stored in the memory 150 to perform or control various operations or functions of the ultrasound imaging device 40. Also, the processor 120 may receive a control signal from the input interface 170 or an external device to control an operation of the ultrasound imaging device 40.

The ultrasound imaging device 40 may include a communication module 160, and be connected to and communicate with an external device (for example, the probe 20, a server, a medical device, or a portable device (a smart phone, a tablet PC, a wearable device, etc.)) through the communication module 160.

The communication module 160 may include one or more components for enabling communication with an external device. The communication module 160 may include at least one of, for example, a short-range communication module, a wired communication module, or a wireless communication module.

The communication module 160 may receive a control signal or data from an external device. The processor 120 may control an operation of the ultrasound imaging device 40 according to a control signal received through the communication module 160. Also, the processor 120 may transmit a control signal to the external device through the communication module 160 and control the external device according to the transmitted control signal. The external device may operate according to the control signal received from the ultrasound imaging device 40, or process data received from the ultrasound imaging device 40.

A program or application related to the ultrasound imaging device 40 may be installed in the external device. The program or application installed in the external device may control the ultrasound imaging device 40, or operate according to a control signal or data received from the ultrasound imaging device 40.

The external device may receive or download the program or application related to the ultrasound imaging device 40 from the ultrasound imaging device 40, the probe 20, or a server, install the program or application therein, and execute the program or application. The ultrasound imaging device 40, the probe 20, or the server, which provides the program or application, may include a recording medium that stores an instruction, a command, an installation file, an execution file, or related data of the corresponding program or application. The external device may be sold with the program or application installed.

The memory 150 may store various data or programs for driving and controlling the ultrasound imaging device 40, input/output ultrasound data, and ultrasound images.

The input interface 170 may receive a user input for controlling the ultrasound imaging device 40. For example, the user input may include an input of operating a button, a key pad, a mouse, a trackball, a jog switch, a knop, etc., an input of touching a touch pad or a touch screen, a voice input, a motion input, a biometric information input (for example, iris recognition, fingerprint recognition, etc.), etc., although not limited thereto.

FIG. 1B is a control block diagram of the ultrasound imaging system 100 with the probe 20 which is a wireless probe or a hybrid probe.

According to various embodiments of the disclosure, the ultrasound imaging device 40 shown in FIG. 1B may be replaced with the ultrasound imaging device 40 described with reference to FIG. 1A.

According to various embodiments of the disclosure, the probe 20 shown in FIG. 1A may be replaced with the probe 20 which will be described with reference to FIG. 1B.

The probe 20 may include a display 112, a transmission module 113, a battery 114, a transducer 117, a charging module 116, a reception module 115, an input interface 109, a producer 118, and a communication module 119. FIG. 1B shows a case in which the probe 20 includes both the transmission module 113 and the reception module 115. However, the probe 20 may include a part of components of the transmission module 113 and the reception module 117, and another part of the components of the transmission module 113 and the reception module 117 may be included in the ultrasound imaging device 40. Also, according to an embodiment of the disclosure, the probe 20 may further include the imaging processor 130.

The transducer 117 may include a plurality of transducers. The plurality of transducers may be arranged in a preset arrangement and implemented as a transducer array. The transducer array may correspond to a 1D array or a 2D array. The plurality of transducers may transmit an ultrasound signal to an object 10 according to a transmission signal applied from the transmission module 113. Also, the plurality of transducers may receive an ultrasound signal reflected from the object 10 and form or generate an electrical reception signal.

The charging module 116 may charge the battery 114. The charging module 116 may receive power from outside. According to an embodiment of the disclosure, the charging module 116 may receive power wirelessly. Also, according to an embodiment of the disclosure, the charging module 116 may receive power by wire. The charging module 116 may transfer the received power to the battery 114.

The processor 118 may control the transmission module 113 to generate or form a transmission signal that is to be applied to each of the plurality of transducers in consideration of positions and focus points of the plurality of transducers.

The processor 118 may control the reception module 115 to perform analog-to-digital conversion on reception signals received from the transducer 117, sum the digital-converted reception signals in consideration of the positions and focus points of the plurality of transducers, and thereby generate ultrasound data. According to an embodiment of the disclosure, the probe 20 may include the imaging processor 130, and in this case, the probe 20 may generate an ultrasound image by using the generated ultrasound data.

In the case in which the probe 20 is implemented as a 2D probe, the processor 118 may calculate a time delay value for digital beamforming for each of a plurality of sub arrays included in a 2D transducer array. Also, the processor 118 may calculate a time delay value for analog beamforming for each of transducers included in any sub array among the plurality of sub arrays. The processor 118 may control an analog beamformer and a digital beamformer to form a transmission signal that is to be applied to each of the plurality of transducers, according to time delay values for analog beamforming and time delay values for digital beamforming. Also, the processor 118 may control the analog beamformer to sum signals received from the plurality of transducers for each sub array according to the time delay values for analog beamforming. Also, the processor 118 may control the ultrasound transmission/reception module 110 to perform analog-to-digital conversion on signals summed for the respective sub arrays. Also, the processor 118 may control the digital beamformer to sum the digital-converted signals according to the time delay value for digital beamforming and generate ultrasound data.

The processor 118 may control overall operations of the probe 20 and control operations of components of the probe 20. The processor 118 may execute a program or instructions stored in the memory 111 to perform or control various operations or functions of the probe 20. Also, the processor 118 may control an operation of the probe 20 by receiving a control signal from the input interface 109 of the probe 20 or an external device (for example, the ultrasound imaging device 40). The input interface 109 may receive a user input for controlling the probe 20. For example, the user input may include an input of operating a button, a key pad, a mouse, a trackball, a jog switch, a knop, etc., an input of touching a touch pad or a touch screen, a voice input, a motion input, a biometric information input (for example, iris recognition, fingerprint recognition, etc.), etc., although not limited thereto.

The display 112 may display an ultrasound image generated by the probe 20, an ultrasound image generated by processing ultrasound data generated by the probe 20, an ultrasound image received from the ultrasound imaging device 40, or various information processed in the ultrasound imaging system 100. Also, the display 112 may further display state information of the probe 20. The state information of the probe 20 may include at least one of device information of the probe 20, battery state information of the probe 20, frequency band information of the probe 20, output information of the probe 20, abnormality information of the probe 20, setting information of the probe 20, or temperature information of the probe 20.

The probe 20 may include a single or plurality of displays 112 according to an implementation type. Also, the display 112 may include a touch panel or a touch screen. Also, the display 112 may include a flexible display.

The communication module 119 may wirelessly transmit the generated ultrasound data or ultrasound image to the ultrasound imaging device 40 through a wireless network. Also, the communication module 119 may receive a control signal and data from the ultrasound imaging device 40.

The ultrasound imaging device 40 may receive ultrasound data or an ultrasound image from the probe 20.

In an embodiment of the disclosure, according to the probe 20 including the imaging processor 130 capable of generating an ultrasound image by using ultrasound data, the probe 20 may transmit ultrasound data or an ultrasound image generated by the imaging processor 130 to the ultrasound imaging device 40.

In an embodiment of the disclosure, according to the probe 20 not including the imaging processor 130 capable of generating an ultrasound image by using ultrasound data, the probe 20 may transmit ultrasound data to the ultrasound imaging device 40. The ultrasound data may include ultrasound raw data, and the ultrasound image may be ultrasound image data.

The ultrasound imaging device 40 may include the processor 120, the imaging processor 130, the display 140, the memory 150, the communication module 160, and the input interface 170.

The imaging processor 130 may generate or process an ultrasound image by using ultrasound data received from the probe 20.

The display 140 may display an ultrasound image received from the probe 20, an ultrasound image generated by processing ultrasound data received from the probe 20, or various information processed in the ultrasound imaging system 100. The ultrasound imaging device 40 may include a single or plurality of displays 140 according to an implementation type. Also, the display 140 may include a touch panel or a touch screen. Also, the display 140 may include a flexible display.

The processor 120 may control overall operations of the ultrasound imaging device 40, and control operations of components of the ultrasound imaging device 40. The processor 120 may execute a program or instruction stored in the memory 150 to perform or control various operations or functions of the ultrasound imaging device 40. Also, the processor 120 may control an operation of the ultrasound imaging device 40 by receiving a control signal from the input interface 170 or an external device.

The ultrasound imaging device 40 may include the communication module 160, and be connected to and communicate with an external device (for example, the probe 20, a server, a medical device, a portable device (a smart phone, a tablet PC, a wearable device, etc.) through the communication module 160.

The communication module 160 may include one or more components for enabling communication with an external device. The communication module 160 may include at least one of, for example, a short-range communication module, a wired communication module, or a wireless communication module.

The communication module 160 of the ultrasound imaging device 40 may communicate with the communication module 119 of the probe 20 through a network or a short-range wireless communication method. For example, the communication module 160 of the ultrasound imaging device 40 may communicate with the communication module 119 of the probe 20 through any one of wireless data communication methods including wireless local area network (LAN), wireless-fidelity (Wi-Fi), Bluetooth, zigbee, Wi-Fi Direct (WFD), infrared communication (infrared Data Association (IrDA)), Bluetooth LowEnergy (BLE), Near Field Communication (NFC), Wireless Broadband Internet (Wibro), World Interoperability for Microwave Access (WiMAX), Shared Wireless Access Protocol (SWAP), Wireless Gigabit Allicance (WiGig), radio frequency (RF) communication, or 60GHz mm Wave short-distance communication.

To this end, the communication module 160 of the ultrasound imaging device 40 and the communication module 119 of the probe 20 may include at least one of a wireless LAN communication module, a Wi-Fi communication module, a Bluetooth communication module, a zigbee communication module, a WFD communication module, an IrDA communication module, a BLE communication module, a NFC communication module, a Wibro communication module, a WiMAX communication module, a SWAP communication module, a WiGig communication module, an RF communication module, or a 60GHz mm Wave short-distance communication module.

In an embodiment of the disclosure, the probe 20 may transmit device information (for example, identification (ID) information) of the probe 20 to the ultrasound imaging device 40 by using a first communication method (for example, BLE) and be wirelessly paired with the ultrasound imaging device 40. Also, the probe 20 may transmit ultrasound data and/or an ultrasound image to the paired ultrasound imaging device 40.

The device information of the probe 20 may include various information related to a serial number, model number, battery state, etc. of the probe 20.

The ultrasound imaging device 40 may receive the device information (for example, ID information) of the probe 20 from the probe 20 by using the first communication method (for example, BLE), and be wirelessly paired with the probe 20. Also, the ultrasound imaging device 40 may transmit an activation signal to the paired probe 20 and receive ultrasound data and/or an ultrasound image from the probe 20. At this time, the activation signal may include a signal for controlling an operation of the probe 20.

In an embodiment of the disclosure, the probe 20 may transmit the device information (for example, ID information) of the probe 20 to the ultrasound imaging device 40 by using the first communication method (for example, BLE), and be wirelessly paired with the ultrasound imaging device 40. Also, the probe 20 may transmit ultrasound data and/or an ultrasound image to the ultrasound imaging device 40 paired by the first communication method by using a second communication method (for example, 60 GHz mm wave or Wi-Fi).

The ultrasound imaging device 40 may receive the device information (for example, ID information) of the probe 20 from the probe 20 by using the first communication method (for example BLE), and be wirelessly paired with the probe 20. Also, the ultrasound imaging device 40 may transmit an activation signal to the paired probe 20, and receive ultrasound data and/or an ultrasound image from the probe 20 by using the second communication method (for example, 60 GHz mm Wave or Wi-Fi).

According to an embodiment of the disclosure, the first communication method used for pairing of the probe 20 and the ultrasound imaging device 40 may have a lower frequency band than that of the second communication method by which the probe 20 transmits ultrasound data and/or an ultrasound image to the ultrasound imaging device 40.

The display 140 of the ultrasound imaging device 40 may display user interfaces (Uls) representing the device information of the probe 20. For example, the display 140 may display ID information of the probe 20 as a wireless ultrasound probe, a pairing method used for pairing with the probe 20, a data communication state between the probe 20 and the ultrasound imaging device 40, a method for data communication with the ultrasound imaging device 40, or a UI representing a battery state of the probe 20.

According to the probe 20 including the display 112, the display 112 of the probe 20 may display a UI representing the device information of the probe 20. For example, the display 112 may display ID information of the probe 20 as a wireless ultrasound probe, a pairing method used for pairing with the probe 20, a data communication state between the probe 20 and the ultrasound imaging device 40, a method for data communication with the ultrasound imaging device 40, or a UI representing a battery state of the probe 20.

The communication module 160 may receive a control signal or data from an external device. The processor 120 may control an operation of the ultrasound imaging device 40 according to a control signal received through the communication module 160.

Also, the processor 120 may transmit a control signal to an external device through the communication module 160 to control the external device according to the control signal. The external device may operate according to the control signal received from the ultrasound imaging device 40 or process data received from the ultrasound imaging device 40.

The external device may receive or download a program or application related to the ultrasound imaging device 40 from the ultrasound imaging device 40, the probe 20, or a server, install the program or application therein, and execute the program or application. The ultrasound imaging device 40, the probe 20, or the server, which provides the program or application, may include a recording medium that stores an instruction, command, installation file, execution file, related data, etc. of the corresponding program or application. The external device may be sold with the program or application installed.

The memory 150 may store various data or programs for driving and controlling the ultrasound imaging device 40, input/output ultrasound data, ultrasound images, etc.

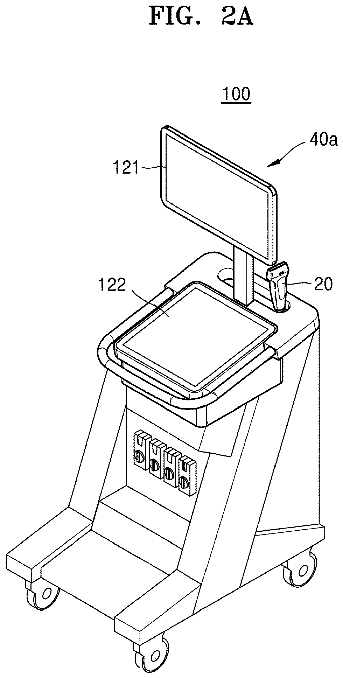

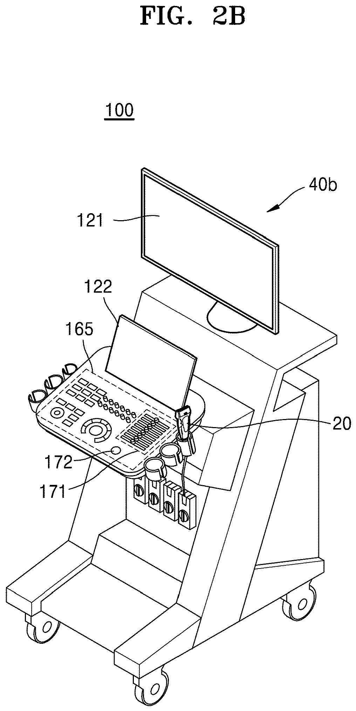

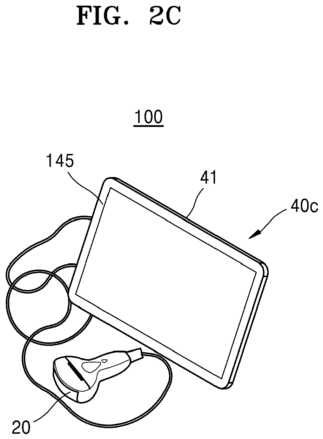

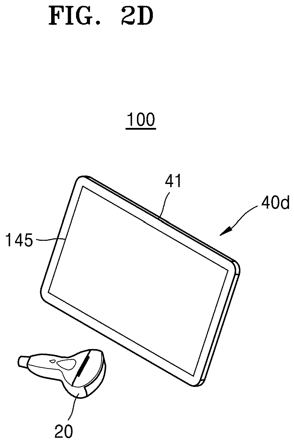

Examples of the ultrasound imaging system 100 according to an embodiment of the disclosure will be described with reference to FIGS. 2A, 2B, 2C, and 2D, below.

FIGS. 2A, 2B, 2C, and 2D show ultrasound imaging devices according to an embodiment of the disclosure.

Referring to FIGS. 2A and 2B, each of ultrasound imaging devices 40a and 40b may include a main display 121 and a sub display 122. The main display 121 and the sub display 122 may correspond to the display 140 of FIGS. 1A and 1B. At least one of the main display 121 or the sub display 122 may be implemented as a touch screen. At least one of the main display 121 or the sub display 122 may display ultrasound images or various information processed in the ultrasound imaging devices 40a and 40b. Also, at least one of the main display 121 or the sub display 122 may be implemented as a touch screen and provide a Graphic User Interface (GUI) to receive data for controlling the ultrasound imaging devices 40a and 40b from a user. For example, the main display 121 may display an ultrasound image, and the sub display 122 may display a control panel for controlling a display of the ultrasound image in the form of a GUI. The sub display 122 may receive control data for controlling a display of an ultrasound image through the control panel displayed in the form of the GUI. For example, a Time Gain Compensation (TGC) button, a Lateral Gain Compensation (LGC) button, a Freeze button, a trackball, a jog switch, or a knop may be provided as a GUI to the sub display 122.

The ultrasound imaging devices 40a and 40b may control the display of the ultrasound image displayed on the main display 121 by using the received control data. Also, the ultrasound imaging devices 40a and 40b may be connected to the probe 20 by wire or wirelessly to transmit/receive an ultrasound signal to/from an object.

Referring to FIG. 2B, the ultrasound imaging device 40b may further include a control panel 165, in addition to the main display 121 and the sub display 122. The control panel 165 may include a button, a trackball, a jog switch, a knop, etc., and receive data for controlling the ultrasound imaging device 40b from a user. For example, the control panel 165 may include a TGC button 171, a Freeze button 172, etc. The TGC button 171 may be a button for setting a TGC value for each depth of an ultrasound image. Also, according to a detection of an input to the Freeze button 171 while the ultrasound imaging device 40b scans an ultrasound image, the ultrasound imaging device 40b may maintain a display state of a frame image at the corresponding time, capture the frame image at the corresponding time, or store the frame image at the corresponding time.

Meanwhile, the button, trackball, jog switch, knop, etc. included in the control panel 165 may be provided as a GUI to the main display 121 or the sub display 122. Also, the ultrasound imaging devices 40a and 40b may be connected to the probe 20 to transmit/receive an ultrasound signal to/from an object.

Also, the ultrasound imaging devices 40a and 40b may include an input/output interface which may be one(s) of various types, such as a speaker, a Light Emitting Diode (LED), a vibrating device, etc. For example, the ultrasound imaging devices 40a and 40b may output various information in the form of graphics, sound, or vibration through the input/output interface. Also, the ultrasound imaging devices 40a and 40b may output various notifications or data through the input/output interface.

Referring to FIGS. 2C and 2D, ultrasound imaging devices 40c and 40d may be implemented as portable types. Examples of the ultrasound imaging devices 40c and 40d which are portable types may include a smart phone, a laptop computer, a PDA, or a tablet PC, which includes a probe and an application, although not limited thereto.

The ultrasound imaging device 40c may include a main body 41. Referring to FIG. 2C, the probe 20 may be connected to one side of the main body 41 by wire. To this end, the main body 41 may include a connection terminal to/from which a cable connected to the probe 20 is attachable/detachable. The probe 20 may include a cable including a connection terminal that is connectable to the main body 41.

Referring to FIG. 2D, the probe 20 may be connected to the ultrasound imaging device 40d wirelessly. The main body 41 may include an input/output interface (for example, a touch screen). The input/output interface may display an ultrasound image, various information processed in the ultrasound image, a GUI, etc.

The ultrasound imaging device 40d may establish communication with the probe 20 or be paired with the probe 20 by using short-range wireless communication. For example, the ultrasound imaging device 40d may communicate with the probe 20 by using Bluetooth, BLE, Wi-Fi, or WFD.

The ultrasound imaging devices 40c and 40d may execute a program or application related to the probe 20 to control the probe 20 and output information related to the probe 20. The ultrasound imaging devices 40c and 40d may perform an operation related to the probe 20 by communicating with a preset server. The probe 20 may be registered in the ultrasound imaging devices 40c and 40d or the preset server. The ultrasound imaging devices 40c and 40d may communicate with the probe 20 registered therein and perform an operation related to the probe 20.

Also, the ultrasound imaging devices 40c and 40d may include an input/output interface which may be one(s) of various types, such as a speaker, a LED, a vibrating device, etc. For example, the ultrasound imaging devices 40c and 40d may output various information in the form of graphics, sound or vibration through the input/output interface. Also, the ultrasound imaging devices 40a and 40b may output various notifications or data through the input/output interface.

According to an embodiment of the disclosure, the ultrasound imaging device 40a, 40b, 40c, or 40d may process an ultrasound image or obtain additional information from an ultrasound image by using an Artificial Intelligence (Al) model. According to an embodiment of the disclosure, the ultrasound imaging device 40a, 40b, 40c, or 40d may generate an ultrasound image or perform processing, such as compensation, image quality enhancement, encoding, or decoding, on an ultrasound image, by using an AI model. Also, according to an embodiment of the disclosure, the ultrasound imaging device 40a, 40b, 40c, or 40d may perform processing, such as defining a base line, obtaining anatomical information, obtaining lesion information, extracting a plane, defining a boundary, measuring a length, measuring a width, measuring a volume, or generating an annotation, on an ultrasound image, by using the AI model.

The AI model may be included in the ultrasound imaging device 40a, 40b, 40c, or 40d or the server.

The AI model may be implemented by using various Artificial Neural Network (ANN) models or Deep Neural Network (DNN) models. Also, the AI model may be learned and generated by using various machine learning algorithms or deep learning algorithms. The AI model may be implemented by using a model, such as, for example, a Convolutional Neural Network (CNN), a Recurrent Neural Network (RNN), a Generative Adversarial Network (GAN), a Long Short-Term Memory (LSTM), etc.

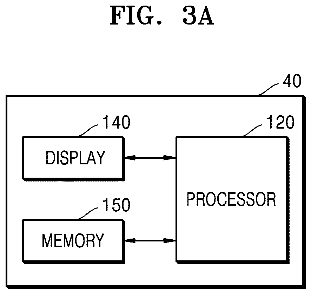

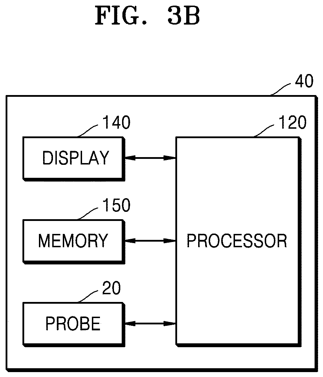

FIGS. 3A and 3B are block diagrams showing configurations of an ultrasound imaging device according to an embodiment of the disclosure. According to various embodiments of the disclosure, an ultrasound imaging device 40 shown in FIGS. 3A and 3B may be replaced with the ultrasound imaging device 40 described with reference to FIGS. 1A and 1B.

Components shown in FIGS. 3A and 3B may be only an embodiment of the disclosure, and components included in the ultrasound imaging device 40 are not limited to those shown in FIGS. 3A and 3B. The ultrasound imaging device 40 according to an embodiment of the disclosure may not include some of the components shown in FIGS. 3A and 3B or may further include another components not shown in FIGS. 3A and 3B. Also, descriptions about ones overlapping with the components shown in FIGS. 3A and 3B will be omitted.

Referring to FIG. 3A, the ultrasound imaging device 40 may include the processor 120, the display 140, and the memory 150.

The display 140 may display each of a plurality of ultrasound images obtained from volume data in a preset display area. In an embodiment, an ultrasound image displayed on the display 140 may include an ultrasound image of which a specific cross-section rotates, or an ultrasound image of which a specific cross-section is converted into another cross-section. In an embodiment, the display 140 may display various indicators related to an ultrasound image together with the ultrasound image. Details about various ultrasound images and indicators that are displayed on the display 140 will be described in more detail through drawings that will be described below and descriptions thereof.

The memory 150 may store instructions or program code for performing functions or operations of the ultrasound imaging device 40. In an embodiment, at least one instruction, algorithm, data structure, program code, and application program stored in the memory 150 may be implemented in a programming or scripting language, such as, for example, C, C++, Java, assembler, etc.

In an embodiment, the memory 150 may include at least one among a flash memory type, a hard disk type, a multimedia card micro type, card type memory (for example, secure digital (SD) memory or extreme digital (XD) memory), Random Access Memory (RAM), Static Random Access Memory (SRAM), Read-Only Memory (ROM), Electrically Erasable Programmable Read-Only Memory (EEPROM), Programmable Read-Only Memory (PROM), Mask ROM, Flash ROM, a Hard Disk Drive (HDD), or a Solid State Drive (SSD).

The processor 120 may execute one or more instructions of a program stored in the memory 150. In an embodiment, the processor 120 may be configured with a hardware component that performs arithmetic, logic, and input/output operations and signal processing. In an embodiment, the processor 120 may be configured with at least one of, for example, a Central Processing Unit (CPU), a microprocessor, a Graphic Processing Unit (GPU), an Application Specific Integrated Circuits (ASICs), Digital Signal Processors (DSPs), Digital Signal Processing Devices (DSPDs), Programmable Logic Devices (PLDs), or Field Programmable Gate Arrays (FPGAs), although not limited thereto. In an embodiment, the processor 120 may include a plurality of processors.

In the case in which a method according to an embodiment of the disclosure includes a plurality of operations, the plurality of operations may be performed by a single or plurality of processors. For example, in the case in which a first operation, a second operation, and a third operation are performed by the method according to an embodiment, all of the first operation, the second operation, and the third operation may be performed by a first processor, or the first operation and the second operation may be performed by the first processor while the third operation may be performed by a second processor. However, the embodiment of the disclosure is not limited thereto.

One or more processors according to the disclosure may be implemented as a single-core processor or a multi-core processor. In the case in which the method according to an embodiment of the disclosure includes a plurality of operations, the plurality of operations may be performed by a single core or by a plurality of cores included in the one or more processors.

Referring to FIG. 3B, the ultrasound imaging device 40 may include the probe 20, the processor 120, the display 140, and the memory 150. Because the processor 120, the display 140, and the memory 150 of the ultrasound imaging device 40 shown in FIG. 3B respectively correspond to the processor 120, the display 140, and the memory 150 of the ultrasound imaging device 40 described with reference to FIG. 3A, overlapping descriptions thereof will be omitted.

The probe 20 may include a wired probe connected to the ultrasound imaging device 40 by wire to communicate with the ultrasound imaging device 40 by wire, a wireless probe wirelessly connected to the ultrasound imaging device 40 to wirelessly communicate with the ultrasound imaging device 40, and/or a hybrid probe connected to the ultrasound imaging device 40 by wire or wirelessly to communicate with the ultrasound imaging device 40 by wire or wirelessly.

In an embodiment, the probe 20 may transmit an ultrasound signal to an object and receive an ultrasound signal reflected from the object, thereby forming a reception signal. In an embodiment, the at least one processor 120 may generate ultrasound data based on the reception signal received from the probe 20. In an embodiment, the at least one processor 120 may control the probe 20 to transmit/receive an ultrasound signal and form a reception signal. Details related to operations of the probe 20 have been described above, and accordingly, overlapping descriptions will be omitted.

Hereinafter, operations of the processor 120 of FIGS. 3A and 3B will be schematically described, and details about each of the operations will be described with reference to the following drawings. Also, the operations of the processor 120 may correspond to operations of the ultrasound imaging device 40 that will be described with reference to the following drawings.

In an embodiment, the at least one processor 120 may execute one or more instructions to obtain a reference cross-sectional image crossing a first axis from volume data.

In an embodiment, the at least one processor 120 may execute one or more instructions to obtain a candidate standard cross-sectional image crossing a second axis that is different from the first axis, from the volume data.

In an embodiment, the at least one processor 120 may execute one or more instructions to display the reference cross-sectional image and the candidate standard cross-sectional image on a first GUI view.

In an embodiment, the at least one processor 120 may execute one or more instructions to rotate the reference cross-sectional image on the first GUI view.

In an embodiment, the at least one processor 120 may execute one or more instructions to adjust a slicing plane of the candidate standard cross-sectional image based on the rotation of the reference cross-sectional image and thereby update the candidate standard cross-sectional image on the first GUI view.

In an embodiment, the at least one processor 120 may include an operation of executing one or more instructions to display, according to completion of the rotation of the reference cross-sectional image, the updated candidate standard cross-sectional image as a standard cross-sectional image on the first GUI view.

In an embodiment, the standard cross-sectional image may include at least one anatomical landmark.

In an embodiment, the standard cross-sectional image may correspond to a Mid-Sagittal Plane (MSP) of an object.

In an embodiment, the reference cross-sectional image may correspond to at least one of a coronal plane or an axial plane of the volume data. In an embodiment, the candidate standard cross-sectional image may correspond to a sagittal plane of the volume data.

In an embodiment, the at least one processor 120 may execute one or more instructions to display a first indicator representing a first line formed as a cross line of a slicing plane of the reference cross-sectional image and a slicing plane of the candidate standard cross-sectional image and a second indicator representing a second line formed as a cross line of the slicing plane of the reference cross-sectional image and a slicing plane of the standard cross-sectional image, on the first GUI view.

In an embodiment, the at least one processor 120 may execute one or more instructions to display an angle indicator representing an angle between the first indicator and the second indicator, on the first GUI view.

In an embodiment, the at least one processor 120 may execute one or more instructions to adjust the slicing plane of the candidate standard cross-sectional imagesuch that the angle between the first indicator and the second indicator corresponds to an angle between the reference cross-sectional image and the standard cross-sectional image.

In an embodiment, the at least one processor 120 may execute one or more instructions to complete rotating the reference cross-sectional image when the angle between the first indicator and the second indicator becomes zero.

In an embodiment, the at least one processor 120 may execute one or more instructions to display at least one anatomical landmark with a preset color that is distinguished from other areas, on the first GUI view.

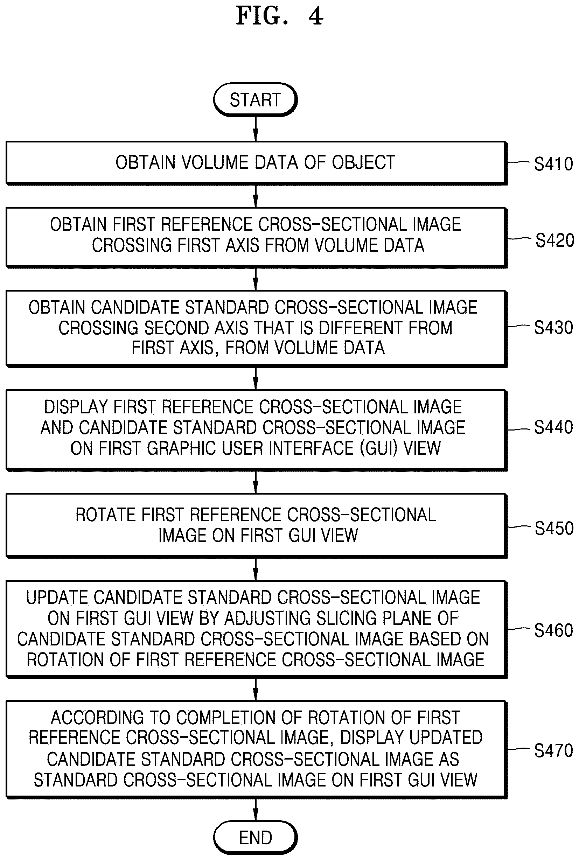

FIG. 4 is a flowchart for describing a method of controlling an ultrasound imaging device, according to an embodiment of the disclosure.

Operations of the ultrasound imaging device 40 will be schematically described with reference to FIG. 4, and details about each of the operations will be described with reference to the following drawings. Operations of the ultrasound imaging device 40, which will be described below, may be performed by the at least one processor 120 of FIGS. 3A and 3B.

In operation S410, the ultrasound imaging device 40 may obtain volume data of an object. The volume data may include three-dimensional ultrasound data formed based on a reception signal received from the probe 20. The volume data may be obtained by three-dimensionally arranging two-dimensional ultrasound images obtained based on the reception signal received from the probe 20 according to positions and performing interpolation, and the volume data may be configured as a group of a plurality of pixels with brightness values located in three dimensions. In an embodiment, the ultrasound imaging device 40 may obtain a two-dimensional ultrasound cross-sectional image by extracting a part of the plurality of pixels of the volume data.

In an embodiment, the ultrasound imaging device 40 may obtain a standard cross-section including at least one anatomical landmark for the object from the volume data.

In an embodiment, the anatomical landmark may be an anatomical feature that is observable in an ultrasound image of the object, and may be used to extract a standard cross-section from the volume data. For example, the anatomical landmark for the object may include a Midline (ML), a choroid plexus (CP), a Diencephalon (DCP), a Nuchal Translucency (NT), a Nasal Bone (NB), and a Palate (PL), although not limited thereto.

In an embodiment, the standard cross-sectional image may include a cross-section required to diagnose the object's condition or disease among ultrasound images. For example, according to an object being a fetus, to observe a growth state according to the number of weeks of the fetus, it may be needed to observe a Crown Rump Length (CRL), which is a length from the top of the fetus's head to the butt. In this case, to more accurately observe the fetus's CRL, it may be needed to obtain the fetus's MSP which is a cross-section including the CRL. However, the disclosure is not necessarily limited to the above-described example. The standard cross-sectional image may include a Para-Sagittal plane (PSP), a Trans-Thalamic Plane (TTP), a Trans-Ventricular Plane (TVP), a Trans-Cerebellar Plane (TCP), etc., and the standard cross-sectional images may include common anatomical landmarks and different anatomical landmarks.

In an embodiment, the ultrasound imaging device 40 may obtain a standard cross-sectional image for the object from the volume data based on a component vector of the anatomical landmark. In an embodiment, the ultrasound imaging device 40 may identify an area corresponding to the anatomical landmark included in the volume data by performing segmentation on the volume data. In an embodiment, the ultrasound imaging device 40 may obtain the component vector of the anatomical landmark by performing Principal Component Analysis (PCA) on the identified area corresponding to the anatomical landmark. In an embodiment, the ultrasound imaging device 40 may identify a center point (or a center of mass) of the area corresponding to the anatomical landmark from the volume data. In an embodiment, the ultrasound imaging device 40 may obtain a standard cross-sectional image including the anatomical landmark by slicing the volume data along a plane that includes the identified center point and is perpendicular to the obtained component vector.

In an embodiment, the ultrasound imaging device 40 may identify the area corresponding to the anatomical landmark from the volume data by applying the volume data to a segmentation module. The segmentation module may include a model learned to output a segmentation map by using volume data as an input. The segmentation map may include data in which a plurality of anatomical landmarks included in the volume data are distinguished from each other. For example, the segmentation map may include data in which pixels not corresponding to the anatomical landmarks in the volume data have a value of 0 and pixels respectively corresponding to the plurality of anatomical landmarks have values other than 0. The segmentation module may be trained based on a learning data set in which a plurality of anatomical landmarks included in volume data are labeled to be distinguished from each other, and by performing training, neural network parameters of the segmentation module may be optimized. In an embodiment, the segmentation module may be stored in the memory 150 of the ultrasound imaging device 40, and the ultrasound imaging device 40 may train the segmentation module stored in the memory 150, or identify an area corresponding to an anatomical landmark through the trained segmentation module stored in the memory 150.

In an embodiment, the ultrasound imaging device 40 may obtain the standard cross-sectional image including the anatomical landmark from the volume data by applying the volume data to a standard cross-sectional image extraction module. The standard cross-sectional image extraction module may include a model learned to extract a standard cross-sectional image including an anatomical landmark by using volume data as an input. For example, according to the volume data being input to the standard cross-sectional image extraction module, at least one among a plurality of standard cross-sectional images that are obtainable from the volume data may be output. In an embodiment, the ultrasound imaging device 40 may obtain a user input of selecting at least one from among the plurality of standard cross-sectional images, and obtain the selected at least standard cross-sectional image from the standard cross-sectional image extraction module. The standard cross-sectional image extraction module may be trained based on a learning data set including volume data and a plurality of standard cross-sectional images that are obtainable from the volume data, and by performing training, neural network parameters of the standard cross-sectional image extraction module may be optimized. In an embodiment, the standard cross-sectional image extraction module may be stored in the memory 150 of the ultrasound imaging device 40, and the ultrasound imaging device 40 may obtain a standard cross-sectional image by training the standard cross-sectional image extraction module stored in the memory 150 or through a trained standard cross-sectional image extraction module stored in the memory 150.

In an embodiment, the ultrasound imaging device 40 may translate the obtained volume data such that a center point of the volume data is located at a center point of the anatomical landmark included in the standard cross-section. In an embodiment, the ultrasound imaging device 40 may translate the volume data by calculating a translation vector based on a difference between the center point of the volume data and the center point of the anatomical landmark included in the standard cross-sectional image and moving coordinates of a plurality of voxels included in the volume data by the calculated translation vector.

In an embodiment, a reference cross-sectional image obtained in operation S420 which will be described below and a candidate standard cross-sectional image obtained in operation S430 which will be described below may be cross-sectional images obtained from the translated volume data. In operation S420, the ultrasound imaging device 40 may obtain a reference cross-sectional image crossing a first axis from the volume data.

In an embodiment, the cross-sectional image crossing the first axis may include a cross-sectional image of the volume data, forming a preset angle with respect to the first axis. For example, in the case in which the first axis is an X axis of a XYZ coordinate system, a reference cross-sectional image crossing the X axis may include a cross-sectional image of the volume data, sliced in a YZ plane that is perpendicular to the X axis. In an embodiment, the ultrasound imaging device 40 may obtain the reference cross-sectional image crossing the X axis by extracting pixels of the volume data, located on a plane crossing the first axis.

In an embodiment, the ultrasound imaging device 40 may obtain the reference cross-sectional image crossing the first axis from the translated volume data. In this case, a location of the center point of the translated volume data may be identical to a location of the center point of the anatomical landmark included in the standard cross-sectional image. Accordingly, the reference cross-sectional image may include the anatomical landmark included in the standard cross-sectional image. For example, in the case in which a standard cross-sectional image is a MSP including a ML of an object, a reference cross-sectional image may include a center point of the ML of the object, and include a cross-section of the ML located on a slicing plane crossing the first axis. In operation S430, the ultrasound imaging device 40 may obtain a candidate standard cross-sectional image crossing a second axis that is different from the first axis, from the volume data.

In an embodiment, the second axis that is different from the first axis may be an axis forming a preset angle with respect to the first axis. For example, in the case in which the first axis is the X axis of the XYZ coordinate system, the second axis may include an Y or Z axis that is perpendicular to the X axis. In an embodiment, the ultrasound imaging device 40 may obtain the candidate standard cross-sectional image by extracting pixels located on a plane crossing the second axis from the volume data.

In an embodiment, because the first axis of the reference cross-sectional image and the second axis of the candidate standard cross-sectional image form the preset angle, the reference cross-sectional image and the standard cross-sectional image may also form the preset angle. In other words, the reference cross-sectional image and the candidate standard cross-sectional image may include anatomical features of the object, which are observable from the volume data sliced along different planes.

In an embodiment, the ultrasound imaging device 40 may obtain a plurality of cross-sectional images crossing the X axis, the Y axis, and the Z axis from the volume data. The ultrasound imaging device 40 may obtain one of the plurality of cross-sectional images as a candidate standard cross-sectional image, and obtain at least one of the remaining cross-sectional images of the plurality of cross-sectional images, not obtained as the candidate standard cross-sectional image, as a reference cross-sectional image. Herein, the plurality of cross-sectional images crossing the X-axis, the Y-axis, and the Z-axis may correspond to a coronal plane, an axial plane, and a sagittal plane, respectively. However, the disclosure is not limited thereto, and the correspondence between the plurality of cross-sectional images crossing the X-axis, Y-axis, and Z-axis and the coronal, axial, and sagittal planes may be different.

In an embodiment, the ultrasound imaging device 40 may obtain the candidate standard cross-sectional image crossing the second axis from the translated volume data. In this case, a location of the center point of the translated volume data may be identical to a location of the center point of the anatomical landmark included in the standard cross-sectional image. Accordingly, the candidate standard cross-sectional image may include the anatomical landmark included in the standard cross-sectional image. For example, in the case in which a standard cross-sectional image is a MSP including a ML of an object, a reference cross-sectional image may include a center point of the ML of the object, and include a cross-section of the ML located on a slicing plane crossing the second axis.

In an embodiment, the ultrasound imaging device 40 may determine, as a candidate standard cross-sectional image, a cross-sectional image corresponding to the standard cross-sectional image from among the coronal plane, the axial plane, and the sagittal plane obtained from the volume data. In an embodiment, each of a plurality of standard cross-sectional images that are obtainable from volume data may have been set in advance to correspond to one among a coronal plane, an axial plane, and a sagittal plane. For example, standard cross-sectional images of a MSP and a PSP may have been set in advance to correspond to a sagittal plane, and standard cross-sectional images of a TTP, a TVP and a TCP may have been set in advance to correspond to an axial plane. In an embodiment, information representing preset correspondences may be stored in the ultrasound imaging device 40, and the ultrasound imaging device 40 may determine one among the coronal plane, the axial plane, and the sagittal plane obtained from the volume data, as a candidate standard cross-sectional image, based on the stored information, and determine at least one of cross-sectional images not determined as the candidate standard cross-sectional image, as a reference cross-sectional image.

In an embodiment, the ultrasound imaging device 40 may calculate an angle of each of the coronal plane, the axial plane, and the sagittal plane obtained from the volume data with respect to the standard cross-sectional image and determine a cross-sectional image having a smallest angle with respect to the standard cross-sectional image, as the candidate standard cross-sectional image. For example, in the case in which a standard cross-sectional image is a MSP, the sagittal plane among the coronal plane, the axial plane, and the sagittal plane may be a cross-sectional image having a smallest angle with respect to the standard cross-sectional image. Accordingly, the ultrasound imaging device 40 may determine the sagittal plane as the candidate standard cross-sectional image, and determine at least one of the coronal plane or the axial plane as the reference cross-sectional image.

In operation S440, the ultrasound imaging device 40 may display the reference cross-sectional image and the candidate standard cross-sectional image on a first GUI view. In an embodiment, the first GUI may include an area on which the reference cross-sectional image is displayed and an area on which the candidate standard cross-sectional image is displayed. In an embodiment, the area on which the candidate standard cross-sectional image is displayed may be larger than the area on which the reference cross-sectional image is displayed.

In an embodiment, a plurality of reference cross-sectional images may be obtained, and in this case, the first GUI may display the plurality of reference cross-sectional images on the first GUI view by including areas on which the plurality of reference cross-sectional images are respectively displayed. For example, reference cross-sectional images may correspond to a coronal plane and an axial plane, and a candidate standard cross-sectional image may correspond to a sagittal plane. In this case, all of the coronal plane, the axial plane, and the sagittal plane obtained from volume data may be displayed on the first GUI view.

In an embodiment, the ultrasound imaging device 40 may display a first indicator representing a first line formed as a cross line of a slicing plane of the reference cross-sectional image and a slicing plane of the candidate standard cross-sectional image and a second indicator representing a second line formed as a cross line of the slicing plane of the reference cross-sectional image and a slicing plane of the standard cross-sectional image, on the first GUI view. In an embodiment, a plurality of reference cross-sectional images may be obtained, and in this case, a first indicator and a second indicator for each of the plurality of reference cross-sectional images may be displayed on the first GUI view. For example, in the case in which the reference cross-sectional images include an axial plane and a coronal plane, a first indicator and a second indicator for the axial plane and a first indicator and a second indicator for the coronal plane may be displayed on the first GUI view.

In an embodiment, because the first indicator represents the first line formed as the cross line of the slicing plane of the reference cross-sectional image and the slicing plane of the candidate standard cross-sectional image, the first indicator may represent position information of the slicing plane of the candidate standard cross-sectional image. Also, because the second indicator represents the first line formed as the cross line of the slicing plane of the reference cross-sectional image and the slicing plane of the standard cross-sectional image, the second indicator may represent position information of the slicing plane of the standard cross-sectional image. Accordingly, the ultrasound imaging device 40 may represent position information of the slicing plane of the candidate standard cross-sectional image and the slicing plane of the standard cross-sectional image with respect to the reference cross-sectional image through the first indicator and the second indicator.

In an embodiment, the ultrasound imaging device 40 may display an angle indicator representing an angle between the first indicator and the second indicator on the first GUI view. In an embodiment, the angle indicator may include a graphics element, such as an icon, a figure, a circular bar, etc., representing an angle, or text representing an angle value. In an embodiment, while a first indicator and a second indicator for each of a plurality of reference cross-sectional images are displayed, an angle indicator may be displayed for each of the plurality of reference cross-sectional images. For example, the ultrasound imaging device 40 may display an angle indicator representing an angle between the first indicator and the second indicator for the axial plane as one of the reference cross-sectional images and an angle indicator representing an angle between the first indicator and the second indicator for the coronal plane, on the first GUI view.

In operation S450, the ultrasound imaging device 40 may rotate the reference cross-sectional image on the first GUI view. In other words, the ultrasound imaging device 40 may display an ultrasound image of which the reference cross-sectional image rotates, on the first GUI view. In an embodiment, in the case in which a plurality of reference cross-sectional images are displayed, the ultrasound imaging device 40 may rotate each of the plurality of reference cross-sectional images on the first GUI view.

In an embodiment, the plurality of reference cross-sectional images may rotate such that a cross-sectional image rotates and then the remaining cross-sectional image rotates. For example, in the case in which the plurality of reference cross-sectional images include a coronal plane and an axial plane, the axial plane may not rotate while the coronal plane rotates on the first GUI view, and after the rotation of the coronal plane is completed, the axial plane may rotate, although not limited thereto. However, all of the plurality of reference cross-sectional images may rotate.

In an embodiment, the ultrasound imaging device 40 may rotate the reference cross-sectional image such that the angle between the first indicator and the second indicator is reduced. In an embodiment, while the reference cross-sectional image rotates, the first indicator may be displayed at a position where the first indicator has previously been displayed. In other words, although the reference cross-sectional image rotates, a display position of the first indicator may not change. In an embodiment, the second indicator may be displayed at a fixed position on the reference cross-sectional image. In other words, while the reference cross-sectional image rotates, the second indictor may be displayed while rotating together with the reference cross-sectional image. Accordingly, while the reference cross-sectional image rotates, the angle between the first indicator and the second indicator may change, and the ultrasound imaging device 40 may rotate the reference cross-sectional image in a direction in which the angle between the first indicator and the second indicator is reduced. In an embodiment, while the reference cross-sectional image rotates, an angle indicator representing the angle between the first indicator and the second indicator may be updated to represent the changed angle between the first indicator and the second indicator and displayed.

In operation S460, the ultrasound imaging device 40 may update the candidate standard cross-sectional image on the first GUI view by adjusting the slicing plane of the candidate standard cross-sectional image based on the rotation of the reference cross-sectional image. Herein, updating the candidate standard cross-sectional image by the ultrasound imaging device 40 may be an operation of obtaining a new candidate standard cross-sectional image by adjusting the slicing plane of the previously obtained candidate cross-sectional image with respect to the volume data and displaying the obtained candidate standard cross-sectional image on the first GUI view.

In an embodiment, the ultrasound imaging device 40 may calculate an angle between the first indicator and the second indicator, changed based on the rotation of the reference cross-sectional image, and adjust the slicing plane of the candidate standard cross-sectional image such that the calculated angle corresponds to an angle between the first line formed as the cross line between the slicing plane of the reference cross-sectional image and the slicing plane of the candidate standard cross-sectional image and the second line formed as the cross line between the slicing plane of the reference cross-sectional image and the slicing plane of the standard cross-sectional image. For example, in the case in which the angle between the first indicator and the second indicator changes from 20 degrees to 10 degrees according to a rotation of the reference cross-sectional image, the ultrasound imaging device 40 may adjust the slicing plane of the candidate standard cross-sectional image such that the angle between the first line and the second line changes from 20 degrees to 10 degrees.

In an embodiment, in the case which a plurality of reference cross-sectional images are obtained, an angle between a first indicator and a second indicator of a reference cross-sectional image that does not rotate may not change. In this case, the ultrasound imaging device 40 may adjust the slicing plane of the candidate standard cross-sectional image such that an angle between a first line formed as a cross line of a slicing plane of the reference cross-sectional image that does not rotate and the slicing plane of the candidate standard cross-sectional image and a second line formed as a cross line of the slicing plane of the reference cross-sectional image that does not rotate and a slicing plane of a standard cross-sectional image does not change.

In operation S470, according to completion of the rotation of the reference cross-sectional image, the ultrasound imaging device 40 may display the updated candidate standard cross-sectional image as a standard cross-sectional image on the first GUI view.

In an embodiment, when the angle between the first indicator and the second indicator becomes zero, the ultrasound imaging device 40 may complete rotating the reference cross-sectional image. In an embodiment, in the case in which a plurality of reference cross-sectional images are obtained, the ultrasound imaging device 40 may complete rotating the reference cross-sectional images when all angles between first indicators and second indicators of the reference cross-sectional images become zero, and display an updated candidate standard cross-sectional image as a standard cross-sectional image on the first GUI view.

In an embodiment, according to completion of the rotation of the reference cross-sectional image, the ultrasound imaging device 40 may display a notification indicator indicating that a standard cross-sectional image has been displayed, on the first GUI view. In an embodiment, the notification indicator may include identification information of the standard cross-sectional image such as a name of the standard cross-sectional image, or include anatomical feature information representing names, sizes, shapes, etc. of anatomical landmarks included in the standard cross-sectional image. Accordingly, a user may easily understand that the rotation of the reference cross-sectional image has currently been completed and the standard cross-sectional image is displayed, through the displayed notification indicator.

In an embodiment, the ultrasound imaging device 40 may display one or more anatomical landmarks with a preset color that is distinguished from other areas, on the first GUI view. In an embodiment, the one or more anatomical landmarks may include one or more anatomical landmarks included in the reference cross-sectional image, the candidate cross-sectional image, and the standard cross-sectional image. In other words, the ultrasound imaging device 40 may display the reference cross-sectional image, the candidate standard cross-sectional image, and the standard cross-sectional image including the anatomical landmarks having the preset color that is distinguished from the other areas, on the first GUI view.

In an embodiment, the one or more anatomical landmarks may be displayed with different preset colors. For example, a ML among the anatomical landmarks may be displayed with a green color on the first GUI view, and a CP may be displayed with a blue color on the first GUI view. As such, because the ultrasound imaging device 40 displays one or more landmarks included in an ultrasound image with different colors, the ultrasound imaging device 40 may help a user analyze anatomical features of the corresponding object.

In an embodiment, the ultrasound imaging device 40 may display one or more anatomical landmarks included in a reference cross-sectional image, a candidate standard cross-sectional image, and a standard cross-sectional image with preset colors by identifying areas corresponding to the anatomical landmarks from volume data. A method in which the ultrasound imaging device 40 identifies an area corresponding to an anatomical landmark included in each cross-sectional image from volume data has been described above, and accordingly, overlapping descriptions will be omitted.

In an embodiment, the ultrasound imaging device 40 may display a three-dimensional ultrasound image of the object on the first GUI view. In an embodiment, the ultrasound imaging device 40 may obtain the three-dimensional ultrasound image of the object by setting a surrounding area of the object to an area-of-interest in the volume data and performing 3D rendering on three-dimensional data set to the area-of-interest in the volume data. For example, the ultrasound imaging device 40 may obtain a three-dimensional ultrasound image of a fetus by setting the fetus's surrounding area to an area-of-interest in volume data and performing 3D rendering.

In an embodiment, the ultrasound imaging device 40 may receive a user input of setting an area-of-interest of the three-dimensional ultrasound image. For example, a user input of setting an area-of-interest may include a touch input of selecting an object of an ultrasound image displayed through the touch screen of the display 140, or a user input of setting an area-of-interest may be received through the input interface 170, although not limited thereto.

In an embodiment, the ultrasound imaging device 40 may change a rendering direction of the three-dimensional ultrasound image based on a rotation of the reference cross-sectional image. In an embodiment, the rendering direction of the three-dimensional ultrasound image may be a direction that is parallel to the slicing plane of the candidate standard cross-sectional image. In an embodiment, by adjusting the slicing plane of the candidate standard cross-sectional image based on the rotation of the reference cross-sectional image, the rendering direction may change to a direction that is parallel to the adjusted slicing plane. In other words, the ultrasound imaging device 40 may update the three-dimensional ultrasound image on the first GUI view by changing the rendering direction of the three-dimensional ultrasound image based on the rotation of the reference cross-sectional image.

As such, the ultrasound imaging device 40 according to an embodiment of the disclosure may visually show a user how much a standard cross-sectional image obtained from volume data is inclined from a reference cross-sectional image, and show the user a process of updating a candidate standard cross-sectional image to a standard cross-sectional image.

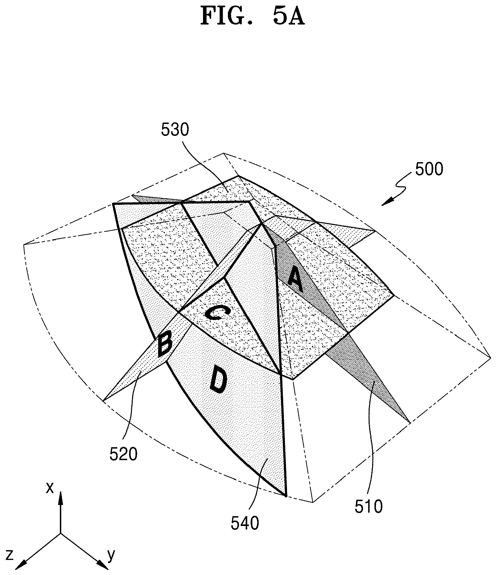

FIG. 5A is a view for describing a reference cross-sectional image and a standard cross-sectional image according to an embodiment of the disclosure.

Referring to FIG. 5A, the ultrasound imaging device 40 may obtain a sagittal plane, an axial plane, and a coronal plane from volume data 500. The sagittal plane may be a cross-sectional image crossing a Z-axis of the volume data 500, and may be a cross-sectional image having an A plane 510 as a slicing plane. The axial plane may be a cross-sectional image crossing a Y-axis of the volume data 500, and may be a cross-sectional image having a B plane 520 as a slicing plane. The coronal plane may be a cross-sectional image crossing an X-axis of the volume data 500, and may be a cross-sectional image having a C plane 530 as a slicing plane. However, the disclosure is not limited thereto, and correspondences of the sagittal plane, the axial plane and the coronal plane to the slicing planes may be different.

In an embodiment, the ultrasound imaging device 40 may obtain a standard cross-sectional image from the volume data 500. The standard cross-sectional image may include at least one anatomical landmark of an object, and may be a cross-sectional image required to diagnose a state or disease of the object and having a D plane 540 of the volume data 500 as a slicing plane. In an embodiment, the standard cross-sectional image having the D plane 540 as the slicing plane may correspond to a MSP of the object.

In an embodiment, the ultrasound imaging device 40 may determine one of the sagittal plane, the axial plane, or the coronal plane as a candidate standard cross-sectional image. For example, the ultrasound imaging device 40 may determine, as the candidate standard cross-sectional image, the A plane 510 of the volume data 500, set in advance to have a correspondence to the standard cross-sectional image. As another example, the ultrasound imaging device 40 may calculate angles of the sagittal plane, the axial plane, and the coronal plane with respect to the standard cross-sectional image, and determine the sagittal plane having a smallest angle as the candidate standard cross-sectional image. Herein, the angles of the sagittal plane, the axial plane, and the coronal plane with respect to the standard cross-sectional image may include angles of the A plane 510, the B plane 520, and the C plane 530 with respect to the D plane 540.

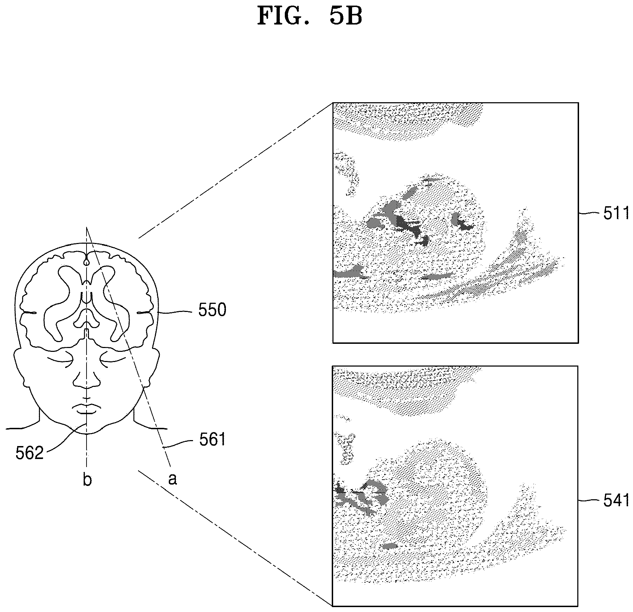

FIG. 5B is a view for describing a candidate standard cross-sectional image and a standard cross-sectional image according to an embodiment of the disclosure.

Referring to FIG. 5B, the ultrasound imaging device 40 may obtain a candidate standard cross-sectional image 511 and a standard cross-sectional image 541. Herein, the candidate standard cross-sectional image 511 may correspond to the sagittal plane having the A plane 510 of FIG. 5A as the slicing plane, and the standard cross-sectional image 541 may correspond to the MSP having the D plane 540 of FIG. 5A as the slicing plane.

In an embodiment, the candidate standard cross-sectional image 511 may be a cross-sectional image that is observable by slicing an object 550 along an a line 561. Herein, the a line 561 may be a line corresponding to the A plane 510 of the volume data 500. In other words, because the A plane 510 of the volume data 500 of the candidate standard cross-sectional image 511 is a preset slicing plane regardless of a position of the object 550, all of one or more landmarks included in the MSP may not be accurately observed in the candidate standard cross-sectional image 511.

According to an embodiment, the standard cross-sectional image 541 may be a cross-sectional image that is observable by slicing the object 550 along a b line 562. Herein, the b line 562 may be a line that makes right and left sides of the object 550 symmetrical. In other words, because the D plane 540 of the volume data 500 of the standard cross-sectional image 541 is a slicing plane determined based on the anatomical landmarks of the object 550, all of the one or more landmarks included in the MSP may be accurately observed in the standard cross-sectional image 541.

As such, the standard cross-sectional image 541 used for diagnosis in the ultrasound imaging device 40 may not be a reference cross-sectional image obtained by crossing the volume data 500 along a preset axis, but a cross-sectional image obtained by extracting to include anatomical landmarks to be observed. In this case, by providing a user with information between the standard cross-sectional image and the reference cross-sectional image, the user's confidence in the standard cross-sectional image may be improved.

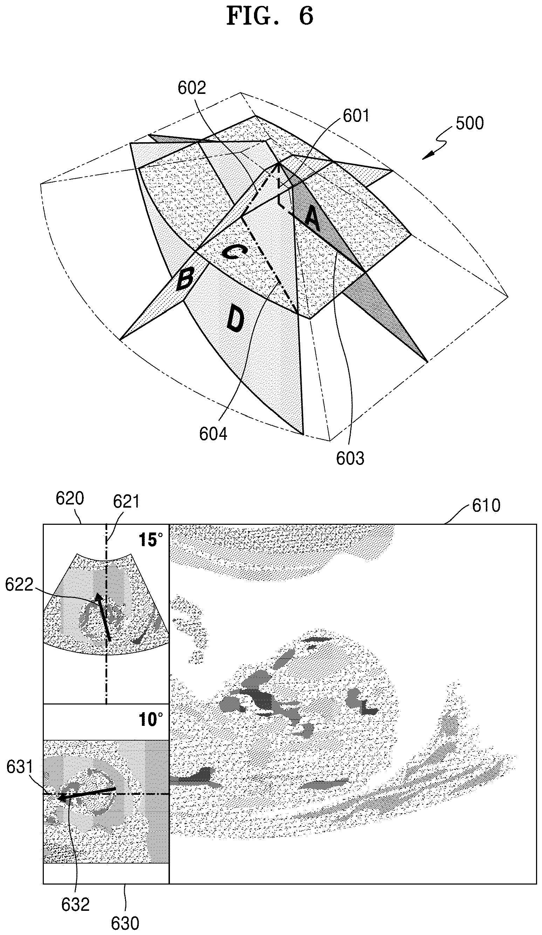

FIG. 6 is a view for describing a first GUI view according to an embodiment of the disclosure.

Referring to FIG. 6, the ultrasound imaging device 40 may display a sagittal plane 610, an axial plane 620, and a coronal plane 630 on a first GUI view. Herein, the first GUI view may include a screen on which the sagittal plane 610, the axial plane 620, and the coronal plane 630 are displayed. Also, the sagittal plane 610 may correspond to a candidate standard cross-sectional image having the A plane 510 of the volume data 500 of FIG. 5A as the slicing plane, and the axial plane 620 and the coronal plane 630 may correspond to reference cross-sectional images having the B plane 520 and the C plane 530 of the volume data 500 of FIG. 5A as the slicing planes.

In an embodiment, the ultrasound imaging device 40 may display a first indicator 621 and a second indicator 622 for the axial plane 620 on the first GUI view. Herein, the first indicator 621 for the axial plane 620 may represent a first line 601 formed as a cross line of the A plane 510 and the B plane 520 on the volume data 500. Also, the second indicator 622 for the axial plane 620 may represent a second line 602 formed as a cross line of the B plane 520 and the D plane 540 as the slicing plane of the MSP on the volume data 500.

In an embodiment, an angle indicator representing an angle of 15 degrees between the first indicator 621 and the second indicator 622 for the axial plane 620 may be displayed on the first GUI view. Herein, the angle of 15 degrees represented by the angle indicator may correspond to an angle between the first line 601 and the second line 602 for the axial plane 620 on the volume data 500.

In an embodiment, the ultrasound imaging device 40 may display a first indicator 631 and a second indicator 632 for the coronal plane 630 on the first GUI view. Herein, the first indicator 631 for the coronal plane 630 may represent a first line 603 formed as a cross line between the A plane 510 and the B plane 530 on the volume data 500. Also, the second indicator 632 for the coronal plane 630 may represent a second line 604 formed as a cross line between the C plane 530 and the D plane 540 as the slicing plane of the MSP on the volume data 500.

In an embodiment, an angle indicator representing an angle of 10 degrees between the first indicator 631 and the second indicator 632 for the coronal plane 630 may be displayed on the first GUI view. Herein, the angle of 10 degrees represented by the angle indicator may correspond to an angle between the first line 603 and the second line 604 for the coronal plane 630 on the volume data 500.

As such, the ultrasound imaging device according to an embodiment of the disclosure may display the first indicators and the second indicators to represent how much the slicing planes of the reference cross-sectional images crossing the volume data along preset axes are inclined with respect to the slicing plane of the standard cross-sectional image. Accordingly, a user may more easily understand position information of the standard cross-section image with respect to the reference cross-sectional images.

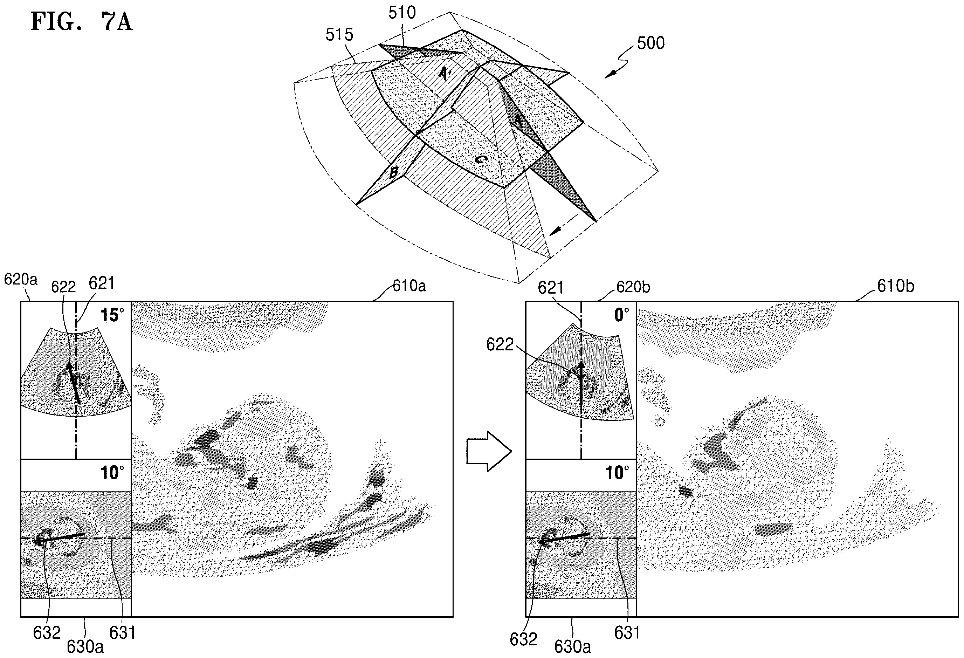

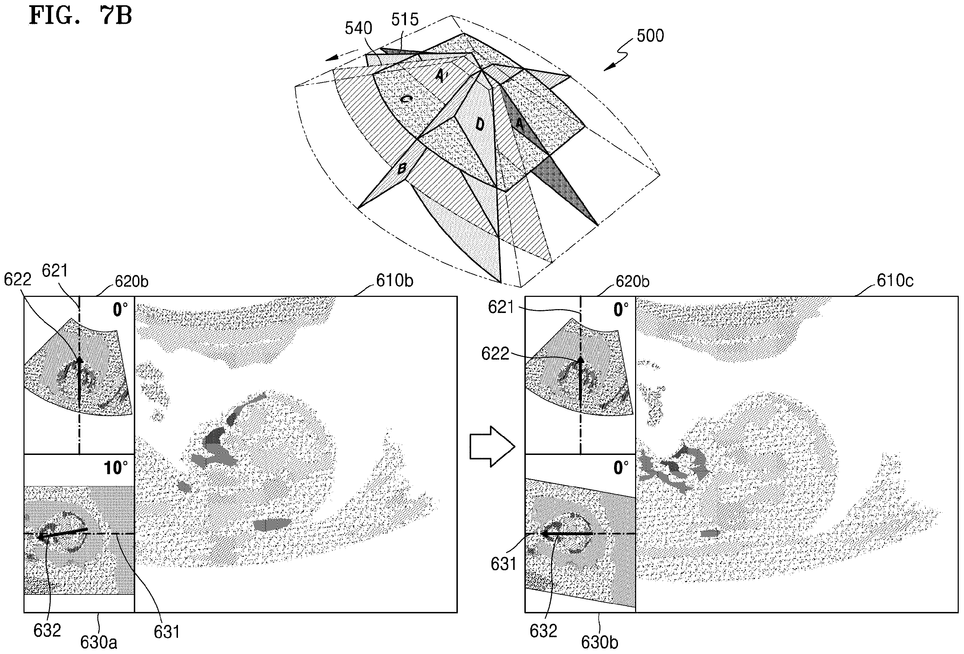

FIGS. 7A and 7B are views for describing an update of a candidate standard cross-sectional image according to an embodiment of the disclosure.

Referring to FIG. 7A, the ultrasound imaging device 40 may rotate an axial plane 620a as one of reference cross-sectional images on a first GUI view. According to completion of the rotation of the axial plane 620a, a rotated axial plane 620b may be displayed. While the axial plane 620a rotates, a first indicator 621 for the axial plane 620a may be displayed at a position at which the first indicator 621 has previously been displayed, and a second indicator 622 for the axial plane 620a may be displayed at a fixed position on the axial plane 620a. Accordingly, an angle between the first indicator 621 and the second indicator 622 may change, and the axial plane 620a may rotate in a clockwise direction in which the angle between the first indicator 621 and the second indicator 622 is reduced.

In an embodiment, when the angle between the first indicator 621 and the second indicator 622 becomes zero, the rotation of the axial plane 620a may be completed. In other words, the axial plane 620a may rotate by 15 degrees in the clockwise direction and then stop. Because the rotated axial plane 620b has rotated until the angle between the first indicator 621 and the second indicator 622 becomes zero, the first indicator 621 and the second indicator 622 displayed together with the rotated axial plane 620b may indicate the same direction.

In an embodiment, while the axial plane 620a rotates on the first GUI view, a coronal plane 630a may not rotate. In other words, the axial plane 620a and the coronal plane 630a as reference cross-sectional images displayed on the first GUI view may rotate independently, and while any one of the axial plane 620a or the coronal plane 630a rotates, another cross-sectional image may not rotate.

In an embodiment, the ultrasound imaging device 40 may adjust a slicing plane of a sagittal plane 610a as a candidate standard cross-sectional image based on the rotation of the axial plane 620a to thereby update the sagittal plane 610a on the first GUI view. In an embodiment, according to completion of the rotation of the axial plane 620a, the sagittal plane 610a having the A plane 510 of the volume data 500 as the slicing plane may be updated to a sagittal plane 610b having an A' plane 515 of the volume data 500 as a slicing plane, and the updated sagittal plane 610b may be displayed on the first GUI view.

While the axial plane 620a rotates, a plurality of axial planes segmented according to preset time intervals or preset angle intervals may be successively displayed. In this case, the slicing plane of the sagittal plane 610a may be adjusted in a direction of gradually approaching the A' plane 515 of the volume data 500 from the A plane 510 of the volume data 500 to respectively correspond to the plurality of axial planes displayed.

In an embodiment, a line formed as a cross line of the A' plane 515 and the B plane 520 of the volume data 500 may correspond to the second line 602 formed as the cross line of the D plane 540 and the B line 520 of the volume data 500 of FIG. 5A. In an embodiment, the slicing plane of the sagittal plane 610a may be adjusted in a direction that is parallel to the B plane 520 as the slicing plane of the axial plane 620a. Accordingly, although the slicing plane of the sagittal plane 610a is adjusted from the A plane 510 to the A' plane 515, an angle between a line formed as a cross line between the adjusted slicing plane and the C plane 530 and the second line 604 formed as the cross line between the D plane 540 and the C plane 530 of the volume data 500 shown in FIG. 6 may not change.

Referring to FIG. 7B, the ultrasound imaging device 40 may rotate the axial plane 620a as one of the reference cross-sectional images on the first GUI view. Because the rotation of the coronal plane 630a corresponds to the rotation of the axial plane 620a described above with reference to FIG. 7A, overlapping descriptions will be omitted.

In an embodiment, the ultrasound imaging device 40 may adjust a slicing plane of the sagittal plane 610b as the candidate standard cross-sectional image based on the rotation of the axial plane 620a to thereby update the sagittal plane 610b on the first GUI view. Herein, the sagittal plane 610b of FIG. 7B may correspond to the sagittal plane 610b updated based on the rotation of the axial plane 620a of FIG. 7A. In an embodiment, according to completion of the rotation of the coronal plane 630a, the sagittal plane 610 having the A' plane of the volume data 500 as the slicing plane may be updated to a sagittal plane 610c having the D plane 540 of the volume data 500 as the slicing plane. Then, the updated sagittal plane 610c may be displayed on the first GUI view. Herein, the updated sagittal plane 610c may correspond to a standard cross-sectional image in view of having the D plane 540 of the volume data 500 as the slicing plane.

In an embodiment, the slicing plane of the sagittal plane 610b may be adjusted in a direction of being parallel to the C plane 530 that is the slicing plane of the coronal plane 630a. Accordingly, although the slicing plane of the sagittal plane 610b is adjusted from the A' plane 515 to the D plane 540, an angle between a line formed as a cross line of the adjusted slicing plane and the B plane 520 and the second line 602 formed as the cross line of the D plane 540 and the C plane 530 shown in FIG. 6 may not change.

As such, the ultrasound imaging device according to an embodiment of the disclosure may update the candidate standard cross-sectional image to the standard cross-sectional image by rotating each of the reference cross-sectional images. Herein, because a direction in which a slicing plane of the candidate standard cross-sectional image is adjusted depends on each of the reference cross-sectional images, a user may easily understand how much the standard cross-sectional image is inclined with respect to each of the reference cross-sectional images.

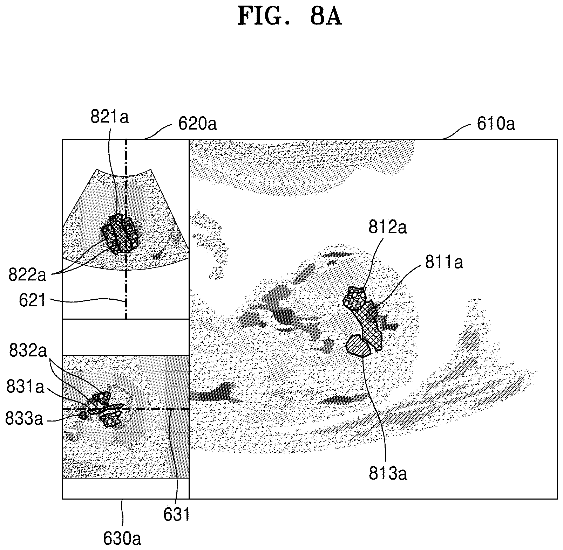

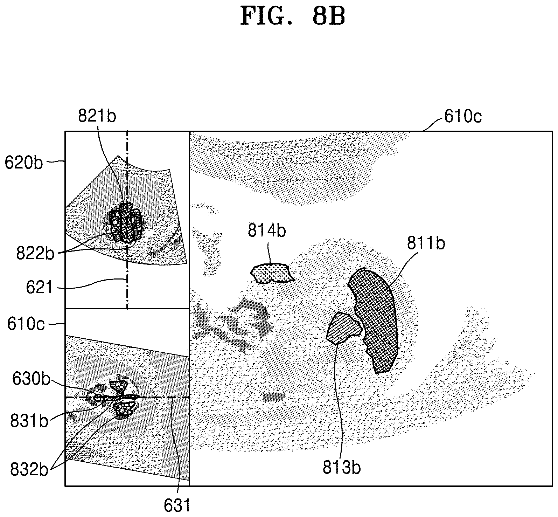

FIGS. 8A and 8B are views for describing displaying of at least one anatomical landmark with a preset color by an ultrasound imaging device according to an embodiment of the disclosure.

Referring to FIG. 8A, the ultrasound imaging device 40 may display at least one anatomical landmark with a preset color that is distinguished from other areas, on the first GUI view. Herein, the sagittal plane 610a of FIG. 8A may correspond to a candidate cross-sectional image, and the axial plane 620a and the coronal plane 630a may correspond to reference cross-sectional images.

In an embodiment, the sagittal plane 610a may include a ML 811a, CP 812a, and DCP 813a of a fetus. In this case, the ML 811a may be displayed with a green color, the CP 812a may be displayed with a blue color, and the DCP 813a may be displayed with a purple color. In an embodiment, in the case in which a standard cross-sectional image is a MSP, the CP 812a may need to be excluded from anatomical landmarks. That is, because the sagittal plane 610a is the candidate standard cross-sectional image before the slicing plane is adjusted based on rotations of the axial plane 620a and the coronal plane 630a, the sagittal plane 610a may not include anatomical features that are observable in the MSP.

In an embodiment, the axial plane 620a may include a ML 821a and CP 822a of the fetus. In this case, the ML 821a of the axial plane 620a may be displayed with a green color, and the CP 822a of the axial plane 620a may be displayed with a blue color.

In an embodiment, the coronal plane 630a may include a ML 831a, CP 832a. and DCP 833a of the fetus. In this case, the ML 831a of the coronal plane 630a may be displayed with a green color, the CP 832a may be displayed with a blue color, and the DCP 833a may be displayed with a purple color.

In an embodiment, the ultrasound imaging device 40 may display first indicators 621 and 631 representing first lines formed as cross lines of slicing planes of the axial plane 620a and the coronal plane 630a as the reference cross-sectional images and the sagittal plane 610a as the candidate standard cross-sectional image, on the first GUI view. In other words, the first indicators 621 and 631 may represent position information of the slicing plane of the sagittal plane 610a, which is observable in the axial plane 620a and the coronal plane 630a. Accordingly, a user may easily understand how much a slicing plane of the MSP as the candidate standard cross-sectional image is inclined with respect to the slicing plane of the sagittal plane 610a by comparing the MLs 821a and 831a displayed with the green color in the axial plane 620a and the coronal plane 630 to the first indicators 621 and 631.

Referring to FIG. 8B, the ultrasound imaging device 40 may display at least one anatomical landmark with a preset color that is distinguished from other areas on a first GUI view. In this case, the sagittal plane 610c of FIG. 8B may correspond to the candidate standard cross-sectional image updated based on the reference cross-sectional images, and the axial plane 620b and a coronal plane 630b may correspond to the reference cross-sectional images completely rotated.

In an embodiment, the sagittal plane 610cmay include a ML 811b, DCP 813b, and NB 814b of the fetus. In this case, the ML 811b and the DCP 813b may be respectively displayed with the same colors as the ML 811a and the DCP 813a of the sagittal plane 610a of FIG. 8A, and the NB 814b may be displayed with a yellow color. In an embodiment, the candidate standard cross-sectional image may be a cross-sectional image updated to a MSP as a standard cross-sectional image by adjusting the slicing plane. Accordingly, the sagittal plane 610c may include the NB 814b which is observable in the MSP, without including the CP 812a, differently from the sagittal plane 610c of FIG. 8A.

In an embodiment, the axial plane 620b may include a ML 821b and CP 822b of the fetus, and the ML 821b and the CP 822b may be respectively displayed with the same colors as the ML 821a and the CP 822a of the axial plane 620a of FIG. 8A.

In an embodiment, the coronal plane 630b may include a ML 831b, CP 832b, and DCP 833b of the fetus, and the ML 831b, the CP 832b, and the CP 833b may be respectively displayed with the same colors as the ML 831a, the CP 832a, and the CP 833a of the coronal plane 630a of FIG. 8A.

As such, according to an embodiment of the disclosure, the one or more anatomical landmarks on the first GUI view may change in size and position while maintaining the preset colors while the candidate standard cross-sectional image is updated according to the rotation of the reference cross-sectional image. Accordingly, a user may more easily recognize a change of anatomical landmarks observable in an image and have more confidence in a standard cross-sectional image displayed on the ultrasound imaging device.

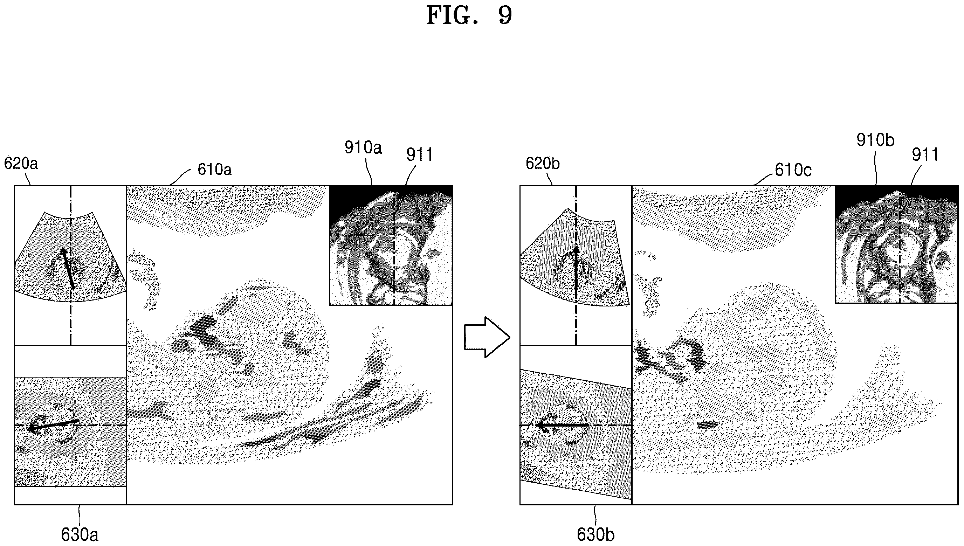

FIG. 9 is a view for describing displaying of a three-dimensional ultrasound image by an ultrasound imaging device according to an embodiment of the disclosure.

Referring to FIG. 9, the ultrasound imaging device 40 may display three-dimensional ultrasound images 910a and 910b of an object on a first GUI view. Sagittal planes 610a and 610c, axial planes 620a and 620b, and coronal planes 630a and 630b of FIG. 9 may respectively correspond to the sagittal planes 610a and 610c, the axial planes 620a and 620b, and the coronal planes 630a and 630b of FIGS. 8A and 8B.

In an embodiment, the ultrasound imaging device 40 may display the three-dimensional ultrasound images 910a and 920b of the fetus by setting a surrounding area of the fetus to an area-of-interest in volume data. In an embodiment, the three-dimensional ultrasound images 910a and 920b of the fetus may be displayed at one areas of the sagittal planes 610a and 61 0b. However, the disclosure is not limited to the above-described example, and ones of the axial planes 620a and 620b and the coronal planes 630a and 630b may be replaced with the three-dimensional ultrasound images 910a and 920b and displayed.

In an embodiment, a rendering direction of the three-dimensional ultrasound images 910a and 920b may be a direction that is parallel to the slicing planes of the sagittal planes 610a and 610b. In an embodiment, the ultrasound imaging device 40 may display a position indicator 911 representing positions of the slicing planes of the sagittal planes 610a and 610b on the three-dimensional ultrasound images 910a and 920b.

For example, on a first GUI view on which the axial plane 620a and the coronal plane 630a not rotated are displayed, the sagittal plane 610a not updated and the three-dimensional ultrasound image 910a having a rendering direction that is parallel to the slicing plane of the sagittal plane 610a may be displayed. In this case, because the sagittal plane 610a is a candidate standard cross-sectional image before the slicing plane is adjusted, the indicator 911 may not be displayed at a location crossing a center of the fetus included in the three-dimensional ultrasound image 910a.

Also, on a first GUI view on which the axial plane 620a and the coronal plane 630a rotated are displayed, the sagittal plane 610c updated to a MSP as a standard cross-sectional image and the three-dimensional ultrasound image 910b having a rendering direction that is parallel to the slicing plane of the sagittal plane 610c may be displayed. In this case, because the sagittal plane 610c corresponds to the MSP, the indicator 911 may be displayed at the location crossing the center of the fetus included in the three-dimensional ultrasound image 910b.

As such, the ultrasound imaging device according to an embodiment of the disclosure may display a three-dimensional ultrasound image of an object together with a cross-sectional image of the object. Herein, because a rendering direction of the three-dimensional ultrasound image can change to correspond to a candidate standard cross-sectional image that is updated to a standard cross-sectional image, a user's confidence in the standard cross-sectional image may be further improved.

Meanwhile, embodiments of the disclosure may be implemented in the form of a computer-readable recording medium including an instruction that is executable by a computer, such as a program module that is executed by a computer. The computer-readable recording medium may be an arbitrary available medium which is able to be accessed by a computer, and may include a volatile or non-volatile medium and a separable or non-separable medium. Further, the computer-readable recording medium may include a computer storage medium and a communication medium. The computer storage medium may include volatile and non-volatile media and separable and non-separable media implemented by an arbitrary method or technology for storing information such as a computer readable instruction, a data structure, a program module, or other data. The communication medium may include other data of modulated data signals, such as computer-readable instructions, data structures, or program modules.

Also, the computer-readable storage media may be provided in the form of a non-transitory storage medium. Herein, the term 'non-transitory storage medium' simply means that the storage medium is a tangible device, and does not include a signal (e.g., an electromagnetic wave), but this term does not differentiate between where data is semi-permanently stored in the storage medium and where the data is temporarily stored in the storage medium. For example, the 'non-transitory storage medium' may include a buffer in which data is temporarily stored.

The aforementioned descriptions are only for illustrative purposes, and it will be apparent that those of ordinary skill in the art can make various modifications thereto without changing the technical spirit and essential features of the disclosure. Thus, it should be understood that the embodiments described above are merely for illustrative purposes and not for limitation purposes in all aspects. For example, each component described as a single type may be implemented in a distributed type, and components described as distributed may be implemented in a combined form.

The scope of the disclosure is indicated by the following claims rather than the detailed description, and all changes or modifications derived from the meaning and scope of the claims and equivalent concepts should be construed as being included in the scope of the disclosure.

## Claims

1. A method of controlling an ultrasound imaging device (40), the method comprising:
obtaining volume data (500) of an object;
obtaining, from the volume data (500), a reference cross-sectional image (620, 630) crossing a first axis;
obtaining, from the volume data (500), a candidate standard cross-sectional image (610) crossing a second axis that is different from the first axis;
displaying the reference cross-sectional image (620, 630) and the candidate standard cross-sectional image (610) on a first graphic user interface (GUI) view;
rotating the reference cross-sectional image (620, 630) on the first GUI view;
updating the candidate standard cross-sectional image (610) on the first GUI view by adjusting a slicing plane of the candidate standard cross-sectional image (610) based on the rotation of the reference cross-sectional image (620, 630); and
displaying, according to completion of the rotation of the reference cross-sectional image (620, 630), the updated candidate standard cross-sectional image (610) as a standard cross-sectional image on the first GUI view,
wherein the standard cross-sectional image includes at least one anatomical landmark.

2. The method of claim 1, wherein the displaying of the reference cross-sectional image and the candidate standard cross-sectional image comprises
displaying a first indicator representing a first line formed as a cross line of a slicing plane of the reference cross-sectional image and the slicing plane of the candidate standard cross-sectional image and a second indicator representing a second line formed as a cross line of the slicing plane of the reference cross-sectional image and a slicing plane of the standard cross-sectional image, on the first GUI view.

3. The method of claim 2, wherein
the displaying of the reference cross-sectional image and the candidate standard cross-sectional image comprises
displaying an angle indicator representing an angle between the first indicator and the second indicator on the first GUI view.

4. The method of claim 2, wherein the rotating of the reference cross-sectional image comprises rotating the reference cross-sectional image such that the angle between the first indicator and the second indicator is reduced.

5. The method of claim 2, wherein the updating comprises adjusting the slicing plane of the candidate standard cross-sectional image such that the angle between the first indicator and the second indicator corresponds to an angle between the reference cross-sectional image and the standard cross-sectional image.

6. The method of claim 5, wherein displaying of the updated candidate standard cross-sectional image as the standard cross-sectional image comprises completing the rotation of the reference cross-sectional image when the angle between the first indicator and the second indicator becomes zero.

7. The method of claim 1, further comprising displaying the at least one anatomical landmark with a preset color that is distinguished from other areas, on the first GUI view.

8. An ultrasound imaging device (40) comprising:
a display (140);
a memory (150) storing one or more instructions; and
at least one processor (120) configured to execute the one or more instructions stored in the memory (150),
wherein the at least one processor (120) is configured to execute the one or more instructions to
obtain volume data of an object,
obtain, from the volume data (500), a reference cross-sectional image (620, 630) crossing a first axis,
obtain, from the volume data (500), a candidate standard cross-sectional image (610) crossing a second axis that is different from the first axis,
display the reference cross-sectional image (620, 630) and the candidate standard cross-sectional image (610) on a first graphic user interface (GUI) view,
rotate the reference cross-sectional image (620, 630) on the first GUI view,
update the candidate standard cross-sectional image (610) on the first GUI view by adjusting a slicing plane of the candidate standard cross-sectional image (610) based on the rotation of the reference cross-sectional image (620, 630), and
display, according to completion of the rotation of the reference cross-sectional image (620, 630), the updated candidate standard cross-sectional image (610) as a standard cross-sectional image on the first GUI view,
wherein the standard cross-sectional image includes at least one anatomical landmark.

9. The ultrasound imaging device of claim 8, wherein the at least one processor is further configured to execute the one or more instructions to
display a first indicator representing a first line formed as a cross line of a slicing plane of the reference cross-sectional image and the slicing plane of the candidate standard cross-sectional image and a second indicator representing a second line formed as a cross line of the slicing plane of the reference cross-sectional image and a slicing plane of the standard cross-sectional image, on the first GUI view.

10. The ultrasound imaging device of claim 9, wherein the at least one processor is further configured to execute the one or more instructions to display an angle indicator representing an angle between the first indicator and the second indicator on the first GUI view.

11. The ultrasound imaging device of claim 9, wherein the at least one processor is further configured to execute the one or more instructions to adjust the slicing plane of the candidate standard cross-sectional image such that the angle between the first indicator and the second indicator corresponds to an angle between the reference cross-sectional image and the standard cross-sectional image.

12. The ultrasound imaging device of claim 8, wherein the at least one processor is further configured to execute the one or more instructions to display the at least one anatomical landmark with a preset color that is distinguished from other areas, on the first GUI view.

13. The ultrasound imaging device of claim 8, wherein the at least one processor is further configured to execute the one or more instructions to display at least one landmark of the object with a preset color.

14. The ultrasound imaging device of claim 8, further comprising
a probe,
wherein the at least one processor is further configured to execute the one or more instructions to obtain the volume data based on a reception signal received from the probe.

15. A computer-readable recording medium storing a program for performing the method of any one of claims 1 to 7 on a computer.
